(19) Europäisches Patentamt

European Patent Office

Office européen des brevets

(11) **EP 2 116 213 B1**

(12) **EUROPEAN PATENT SPECIFICATION**

(45) Date of publication and mention
of the grant of the patent:
**29.03.2017 Bulletin 2017/13**

(21) Application number: **08710721.5**

(22) Date of filing: **01.02.2008**

(51) Int Cl.:
*A61F 2/95* *(2013.01)*     *A61B 17/12* *(2006.01)*
*A61F 2/966* *(2013.01)*     *A61F 2/92* *(2013.01)*
*A61F 2/07* *(2013.01)*

(86) International application number:
**PCT/JP2008/051689**

(87) International publication number:
**WO 2008/093851 (07.08.2008 Gazette 2008/32)**

(54) **MEDICAL DEVICE FOR BODY CAVITY AND METHOD OF PRODUCING THE SAME**

MEDIZINISCHES GERÄT FÜR EINE KÖRPERHÖHLE UND HERSTELLUNGSVERFAHREN DAFÜR

DISPOSITIF MÉDICAL POUR UNE CAVITÉ CORPORELLE ET SON PROCÉDÉ DE PRODUCTION

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **01.02.2007   JP 2007023228**

(43) Date of publication of application:
**11.11.2009   Bulletin 2009/46**

(73) Proprietors:
• **Kaneka Corporation
Osaka (JP)**
• **Gifu University
Gifu-shi
Gifu 501-1194 (JP)**

(72) Inventors:
• **YOSHIMURA, Shinichi
Gifu-shi
Gifu 501-1194 (JP)**
• **HASHIMOTO, Tadaaki
Settsu-shi
Osaka 566-0072 (JP)**
• **FUKAYA, Kohei
Settsu-shi
Osaka 566-0072 (JP)**

(74) Representative: **Gille Hrabal
Brucknerstrasse 20
40593 Düsseldorf (DE)**

(56) References cited:
EP-A1- 0 716 836     EP-A2- 0 819 412
WO-A1-99/48441      WO-A1-2006/071245
WO-A2-2005/055800   JP-A- 07 024 072
JP-A- 08 224 297     JP-A- 11 299 901
US-A1- 2002 173 836   US-A1- 2006 136 042
US-A1- 2006 184 237   US-B1- 6 214 040
US-B2- 6 821 296

**Description**

TECHNICAL FIELD

The present invention relates to a new medical device for treatment of diseases of patient's organs in the lumen structure such as blood vessel, for example for treatment of aneurysm, and, in particular, to a medical device for body cavity for indwelling a sheet-shaped member and an expandable stent in a region close to a lesion such as of aneurysm or carotid artery obstruction, and a method of producing the same. The present invention also relates to a delivery system containing the medical device for body cavity.

BACKGROUND ART

A common method of treating aneurysm formed on vascular wall so far known is to indwell multiple embolic coils in the aneurysm. Specifically, coils are delivered one by one into the aneurysm through a microcatheter by a deployment device. In the case of a wide-necked aneurysm, the embolic coils may migrate into the blood vessel, possibly leading to obstruction of the parent blood vessel, and thus, care should be given to its use.

In the technology described in Patent Document 1 the shape of the pores in the blocking device used varies between during contraction and during expansion, when the blocking device is placed on an aneurysmal opening. The expanded diameter of the blocking device also varies according to the diameter of the blood vessel, and the shape of the pore also varies according to the blood vessel and the disease of the patient even when the same device is used. The shape and the size of the pore cause a difference in the rate of thrombosis by blood inflow, and the shape and the size of the pore exert a great influence on the obstructive action on the aneurysm. Therefore, possibly with the blocking device of Patent Document 1, the curative effect may vary according to the disease and the operation used.

Alternatively, devices in a configuration similar to that described in Patent Document 2 were disclosed, but in these devices, embolic components should be placed reliably in the aneurysmal side during indwelling, and thus, they are still unsatisfactory in the convenience of operation. In addition, aneurysms possibly treated are limited by the size and the shape of the embolic component used. For use in emergency, for example in treatment of ruptured aneurysm, a considerable amount of experience in examination and operation is demanded. In the technologies disclosed in Patent Documents 1 and 2, there was demonstrated no relationship between the obstructive action and the size of the pore in the blocking device.

It is possible by the technology disclosed in Patent Document 3 to cover a lesion with a cover stent having a cover region of a predetermined diameter along the desired blood vessel.

However, actual blood vessel has a blood vessel diameter slightly varying locally in each region. Unfavorably with the stent cover described in the invention of Patent Document 3, it is not possible to bring the cover region into contact with the blood vessel internal wall if the cover region is smaller than the diameter of the blood vessel in which it is to be indwelled, and the stent cover region remains unincorporated, if the diameter of the stent cover is larger than that of the blood vessel. An excessively large stent cover region leads to indwelling of the stent cover as it is folded, resulting in blood flow into the aneurysm through the folded region (because the cover is not in tight contact). Thus, insufficient conformity between the blood vessel diameter and the stent diameter may prohibit tight contact.

In the technology disclosed in Patent Document 4, the cover region cannot be brought into tight contact with the blood vessel internal wall because of the presence of a belt buckle-shaped region, and the blood may possibly flow into the aneurysm, as the cover region is lifted upward.

The technology disclosed in Patent Document 5 teaches that a porous region is needed for mutual fixation of the sheets and conversion to endodermis, and the size and the shape of the pore is not particularly limited, if the pore permits migration of endothelial cells.

In the technology disclosed in Patent Document 6, if a force extending radially from the center of a folded sheet is applied, a structure having sheets folded in the coil shape cannot be expanded, unless the force applied radially (in the direction vertical to the stent circumference) is converted to a force applied to open the coil-shaped sheet (in the direction tangent to the stent circumference). It is also difficult to indwell the sheet, as it is adjusted to a particular direction, because the sheet expands, while moving circularly like a spring and pushing the vascular wall, during expansion of the stent

In the technology disclosed in Patent Document 7, because the stent and the sheet region are separate from each other, there are often undesired phenomena such as revolution of the sheet region during expansion, and thus, it would be difficult to expand the sheet member, as it is directed toward a targeted lesion.

EP 0 819 412 A2 discloses an endoprosthesis assembly which is used on a balloon catheter wherein the assembly includes a expandable stent and a low profile cover which is wrapped over the stent.

Patent Document 1: JP-A No. 2001-509412

Patent Document 2: JP-A No. 2003-175113
Patent Document 3: JP-A No. 2001-506176
Patent Document 4: JP-A No. 8-24346
Patent Document 5: JP-A No. 2005-52419
Patent Document 6: JP-A No. 8-224297 and EP 07 16836 A1
Patent Document 7: U.S. Patent No. 005779732

SUMMARY OF THE INVENTION

Problems to be Solved by the invention

The invention is defined by the claims. Insofar as the terms "invention" and/or "embodiment" are used in the following, and/or features are presented as being optional, this should be interpreted in such a way that the only protection sought is that of the invention as claimed. Methods of using a vascular device are presented as a way to understand the invention and do not form part of the invention.

An object of the present invention is to provide a new medical device for body cavity that covers openings formed in various body lumen structures (celomic cavities) such as aneurysm, varicosis and microaneurysm more efficiently than conventional stents, reduces or blocks the blood flow into the opening distinctively in a short period of time and converts the opening to endodermis rapidly, a method of producing the same, and a delivery system containing the medical device for body cavity.

Means to Solve the Problems

After intensive studies to solve the problems above, the inventors found that a medical device having a sheet-shaped member that spreads following the main stent body along the circumferential direction can block the opening of celomic cavities such as aneurysm efficiently without difficulty in operation, and made the present invention.

Specifically, the present invention (1) relates to a medical device for body cavity, comprising a main stent body and a sheet-shaped member having pores and covering at least part of the main stent body, characterized in that the sheet-shaped member is fixed only to the most distal region of the main stent body at one point.

The present invention (2) relates to the medical device for body cavity above, wherein, when the main stent body is expanded from a compressed first diameter to a second diameter, the sheet-shaped member spreads in such a manner that the sheet-shaped member does not restrict the movement of the main stent body in the circumferential direction and covers at least part of the main stent body, and the shape of the pores of the sheet-shaped member is preserved even after expansion of the main stent body.

The present invention (3) relates to the medical device for body cavity, wherein the sheet-shaped member is a sheet-shaped member having a fine structure and the fine structure of the sheet-shaped member is preserved without change even after expansion of the main stent body. The fine structure is a structure having pores in a particular shape, such as polygon (e.g., quadrangle), circle, or ellipse, formed on the surface of the sheet-shaped member, which is made, for example, of a mesh.

The present invention (4) relates to the medical device for body cavity, comprising a main stent body formed in a tubular structure expandable from a compressed first diameter to a second diameter and a sheet-shaped member having pores and covering at least part of the main stent body, wherein when the main stent body is expanded, the sheet-shaped member the movement of the main stent body in the circumferential direction and covers at least part of the main stent body, and the shape of the pores of the sheet-shaped member is preserved even after expansion of the main stent body.

The present invention (5) relates to the medical device for body cavity, comprising a main stent body formed in a tubular structure and expandable in the circumferential direction from a compressed first diameter to a the second diameter and a fine-structured sheet-shaped member having multiple pores, wherein the sheet-shaped member spreads, as it covers at least part of the main stent body, together with the expansion of the main stent body in the circumferential direction when the main stent body is expanded and the fine structure of the sheet-shaped member is preserved without change even after expansion of the main stent body.

The present invention (7) relates to the medical device for body cavity, wherein the sheet-shaped member is fixed to the region of the main stent body in such a manner that the size of the pores is preserved substantially after expansion of the main stent body.

The present invention (8) relates to the medical device for body cavity, wherein the fixing member is a wire.

The present invention (13) relates to the medical device for body cavity, wherein the sheet-shaped member is fixed to the main stent body region, with a fixing member having an element in parallel with the axial direction of the main stent body region. The present invention (14) relates to the medical device for body cavity, wherein the sheet-shaped member is sewn with a wire.

The present invention (15) relates to the medical device for body cavity, wherein the sheet-shaped member and the main stent body are fixed to each other with a metal or polymeric material.

The present invention (16) relates to the medical device for body cavity, wherein the polymeric material is a polyvinylalcohol (PVA), a cyanoacrylate, a biodegradable material or a glycolic polylactate acid copolymer (PLGA).

The present invention (17) relates to the medical device for body cavity, wherein the main stent body has a length larger than that of the sheet-shaped member.

The present invention (18) relates to the medical device for body cavity, wherein the internal diameter of the main stent body is 0.01 to 0.04 inch (0.25 to 1.02 mm) when it has a compressed first diameter.

The present invention (19) relates to the medical device for body cavity, wherein the main stent body is expanded in the shape of straight tube.

The present invention (20) relates to the medical device for body cavity, wherein the main stent body is expanded into a tapered shape.

The present invention (21) relates to the medical device for body cavity, wherein at least part of the main stent body has a structure for fixation of the sheet-shaped member.

The present invention (22) relates to the medical device for body cavity, wherein the sheet-shaped member contains an X-ray-impermeable filament material at least partially.

The present invention (23) relates to the medical device for body cavity, wherein the sheet-shaped member contains an X-ray-impermeable material at the fixing site.

The present invention (24) relates to the medical device for body cavity, wherein the medical device contains the sheet-shaped member in which the first and second terminals of the sheet-shaped member overlap each other, when the main stent body region has the first diameter.

The present invention (25) relates to the medical device for body cavity, wherein the sheet-shaped member overlaps the circumference of the stent in an angular range of 340° or less.

The present invention (26) relates to the medical device for body cavity, wherein the sheet-shaped member covers the entire circumference of the main stent body region, when the main stent body region has the second diameter.

The present invention (27) relates to the medical device for body cavity, wherein the sheet-shaped member covers the circumference of the stent circumference in an angular range of 60° or more and 300° or less, when the main stent body region has the second diameter.

The present invention (28) relates to the medical device for body cavity, wherein the sheet-shaped member is a mesh.

The present invention (29) relates to the medical device for body cavity, wherein the mesh is formed by weaving a filament material.

The present invention (30) relates to the medical device for body cavity, wherein the wire diameter of the filament material of the sheet-shaped member is smaller than the thickness of the main stent body region.

The present invention (31) relates to the medical device for body cavity, wherein the wire diameter of the filament material is 0.2 mm or less.

The present invention (32) relates to the medical device for body cavity, wherein the size of the pores of the sheet-shaped member is 0.2 mm or less.

The present invention (33) relates to the medical device for body cavity, wherein the pore rate of the sheet-shaped member is 50% or less,

The present invention (34) relates to the medical device for body cavity, wherein the average thickness of the sheet-shaped member is 0.2 mm or less.

The present invention (35) relates to the medical device for body cavity, wherein the material for the sheet-shaped member is a metal.

The present invention (36) relates to the medical device for body cavity, wherein the metal content of the sheet-shaped member is 16.3 mg/mm$^2$ or less.

The present invention (37) relates to the medical device for body cavity, wherein the material for the sheet-shaped member is SUS or SUS316.

The present invention (38) relates to the medical device for body cavity, wherein the medical device for body cavity, wherein the sheet-shaped member is a plain- or twill-woven member.

The present invention (39) relates to the medical device for body cavity, wherein the curvature memorized by the sheet-shaped member is different from the curvature of the main stent body in the second diameter.

The present invention (40) relates to the medical device for body cavity, wherein at least part of the sheet-shaped member has a curvature in a direction different from that of the main stent body in the second diameter.

The present invention (41) relates to the medical device for body cavity, wherein the sheet-shaped member has folding lines at least partially.

The present invention (42) relates to the medical device for body cavity, wherein the sheet-shaped member memorizes waveform.

The present invention (43) relates to the medical device for body cavity, wherein the materials for the main stent body and the sheet-shaped member are the same.

The present invention (44) relates to the medical device for body cavity, wherein the same material for the main stent body and the sheet-shaped member is a metal.

The present invention (45) relates to a method of producing the medical device, comprising a sewing step of making the wire have an angle in the terminal region, placing the sheet-shaped member at a particular position, sending the wire, as it penetrates through the sheet-shaped member and the main stent body from the external surface side to the internal peripheral side of main stent body, and sending the wire terminal region from the stent internal peripheral side to the external surface side by feeding a rod having a diameter similar to the diameter of the stent internal diameter into the internal peripheral side of the main stent body.

The present invention (46) relates to a delivery system, comprising the medical device for body cavity and a first catheter for feeding the medical device for body cavity into the body, wherein the medical device for body cavity is located in the distal region of the first catheter so that the medical device can be indwelled.

The present invention (47) relates to the delivery system, wherein at least part of the sheet-shaped member is placed separately from the main stent body.

The present invention (48) relates to the delivery system, wherein the first catheter has a first tubular member, the main stent body having a compressed first diameter can be placed internally at the distal end of the first tubular member, and the sheet-shaped member can be placed externally at the distal end of the first tubular member.

The present invention (49) relates to the delivery system, wherein the first catheter has a first tubular member forming the catheter main body and a second tubular member placed externally on the first tubular member, and the main stent body having a compressed first diameter can be placed internally at the distal end of the first tubular member and the sheet-shaped member can be placed between the first and second tubular members.

The present invention (50) relates to the delivery system, wherein the first tubular member has at least one penetrating lumen at the distal end.

The present invention (51) relates to the delivery system, wherein the first tubular member has a hole communicating with the lumen at least at one position on the outermost surface.

The present invention (52) relates to the delivery system, wherein the first tubular member has a slit at the distal end.

The present invention (53) relates to the delivery system, wherein the second tubular member is the protective unit of the sheet-shaped member.

The present invention (54) relates to the delivery system, wherein the protective unit is a thin film.

The present invention (55) relates to the delivery system, wherein the thin film is made of a polymer compound material.

The present invention (56) relates to the delivery system, wherein the internal diameter of the protective unit is 1.5 to 2.5 mm.

The present invention (57) relates to the delivery system, wherein the protective unit is fixed to the first tubular member.

The present invention (58) relates to the delivery system, wherein the external shape of the protective unit is a tapered shape.

The present invention (59) relates to the delivery system, wherein the main stent body region having a compressed first diameter is placed in the most internal lumen of the first tubular member and the sheet-shaped region is placed between the first and second tubular members.

The present invention (60) relates to the delivery system, further comprising a second catheter, penetrating inserted in the first tubular member so as to push and for use in pushing the medical device for body cavity from the rear edge side.

The present invention (61) relates to the delivery system, wherein the second catheter is tubular in shape and its internal diameter is 0.01 to 0.04 inch (0.25 to 1.02 mm).

The present invention (62) relates to the delivery system, wherein the first or second catheter retains the internal diameter allowing absorption after indwelling the medical device.

Advantageous Effects of the Invention

The medical device for body cavity according to the present invention covers undesired openings and other lesions of celomic cavity such as aneurysm with a sheet-shaped member having pores. In the present invention, because the sheet-shaped member has particular pores that reduce blood flow through the opening of celomic cavity into aneurysm distinctively and yet assure flow of blood components through the sheet-shaped member, the organ in aneurysm can be regenerated rapidly.

In addition, structurally with the medical device for body cavity according to the present invention, it is possible to reduce the thrombotic debris scattered from the lesion near vascular wall and flowing into the blood vessel, atheromatous plaques, aneurysmal embolic coils sticking out from aneurysm, and others. The medical device for body cavity according to the present invention can also be used as an auxiliary for prevention of damage on fragile affected blood vessels.

Further in the medical device for body cavity according to the present invention, as the sheet-shaped member is fixed to the main stent body as it is folded around it in the circumferential direction, the sheet-shaped member can be spread rapidly without change in diameter and shape of the pores during the period from contraction to expansion of the stent.

In addition, the method of covering an opening with a sheet-shaped structure prepared by using the medical device for body cavity according to the present invention is effective to openings in various sizes generated in celomic cavities different in diameter, and in particular, for blockage of the openings that cannot be treated for example with embolic material or coil and also of large-necked aneurysms, and it is also effective both to unruptured and ruptured aneurysms.

Because the mesh-like sheet-shaped member can be processed to be self-expandable, it becomes possible to perform operation of celomic cavity obstruction more easily by using a delivery system such as that described below than by using a balloon catheter and a flat plate-like sheet-shaped member.

In particular, if the mesh-shaped material is a metal, blood vessel tends to be regenerated rapidly. If the material for the main stent body is a metal, it is easy to produce a self-expanding medical device for body cavity.

It is possible to cover the opening generated in the celomic cavity with the sheet-shaped member, by placing the medical device for body cavity according to the present invention on the balloon surface of a balloon catheter and expanding the main stent body therein by means of inflating the balloon in the targeted celomic cavity region.

Because the sheet-shaped member in the medical device for body cavity according to the present invention is not tubular in shape primarily, it fits to various vascular walls favorably, and thus, can be applied to various blood vessels different in diameter and various openings different in shape.

In addition, it is possible with the delivery system according to the present invention, to indwell a medical device for body cavity comprising a sheet-shaped member and a main stent body easily and safely. When the sheet-shaped member and the main stent body are placed separately, it is possible to place a thin fine sheet-shaped member in the delivery system easily without any plastic deformation such as crinkling.

BRIEF DESCRIPTION OF THE DRAWINGS

Figure 1 is a side view of the medical device in an embodiment of the present invention before expansion.
Figure 2 is views of the medical devices in an embodiment before expansion, as seen from the axial direction.
Figure 3 is a side view of the medical device in an embodiment after expansion.
Figure 4 is a view of the medical device in an embodiment after expansion, as seen from the axial direction.
Figure 5 is a schematic view of a medical device before expansion that is placed at a certain position close to aneurysm.
Figure 6 is a schematic view of a medical device after expansion that is placed at a certain position close to aneurysm.
Figure 7 are schematic views of the sheet-shaped member in an embodiment as seen from fixed face side, when it is fixed to a stent at two points.
Figure 8 is a schematic view of a sheet-shaped member as seen from fixed face side, when it is fixed to a stent at two points.
Figure 9 is a schematic view of a sheet-shaped member as seen from fixed face side, when it is fixed to a stent at two or three points.
Figure 10 is a schematic view of a sheet-shaped member as seen from fixed face side, when it is fixed to a stent at two or three points.
Figure 11 is a schematic view of a sheet-shaped member as seen from fixed face side, when it is fixed to a stent at two to four points.
Figure 12 is a schematic view of a sheet-shaped member as seen from fixed face side, when it is fixed to a stent at two to four points.
Figure 13 is a schematic view of the shape of a sheet-shaped member, when it is fixed to a stent at two or three points, before expansion or during expansion.
Figure 14 is schematic views of the shape of a sheet-shaped member, when it is fixed to a stent at two to four points, before expansion or during expansion.
Figure 15 is a schematic view of a sheet-shaped member, when it is fixed to a stent in a linear shape.

EP 2 116 213 B1

Figure 16 is a schematic view of an elliptic sheet-shaped member.

Figure 17 is a schematic view of a polygonal sheet-shaped member.

Figure 18 is a schematic view of a medical device having a sheet-shaped member internally fixed, after expansion.

Figure 19 is a side view of a medical device in which the sheet-shaped member covers the stent in an angular range of 360°, after expansion.

Figure 20 is slides of pathological samples containing an indwelling sample stent.

Figure 21 shows SEM photographs of a sample stent covered with a twill-woven metal mesh that is placed in an aneurysmal phantom in a blood perfusion test.

Figure 22 shows SEM photographs and a photograph of a sample stent covered with a plain-woven metal mesh that is placed in an aneurysmal phantom after a blood perfusion test.

Figure 23 shows SEM photographs of a sample stent covered with a plain-woven metal mesh that is placed in an aneurysmal phantom in a blood perfusion test.

Figure 24 shows radiographs of a sample stent before and after indwelling and one month after indwelling.

Figure 25 shows schematic explanatory drawings showing a method of producing the medical device according to the present invention.

Figure 26 is a schematic view of a pre-molded sheet-shaped member used in the medical device.

Figure 27 is a schematic explanatory drawing illustrating a delivery system for self-expandable medical device.

Figure 28 is a schematic view illustrating the second catheter used for delivery of the medical device in the delivery system shown in Figure 27.

Figure 29 is a schematic explanatory drawing illustrating the distal region of the delivery system shown in Figure 27.

Figure 30 shows a photograph showing an example of the appearance of the distal region of the delivery system shown in Figure 27.

Figures 31(a) to 31(f) show an example of the delivery system for self-expanding medical device.

Figures 32(a) to 32(f) show another example of the delivery system for self-expanding medical device.

Figure 33 shows radiographic images of a medical device before and after indwelling.

Figure 33(a) shows blood flow in a blood vessel having aneurysm.

Figure 33(b) shows blood flow immediately after indwelling a medical device in the blood vessel above.

Figure 33(c) is a sectional view of the organ including the aneurysmal region and the blood vessel, two months after indwelling the medical device.

Figures 34(a) and 34(b) are radiographs of the area close to an aneurysm synthetically-prepared in canine carotid artery in Example 7.

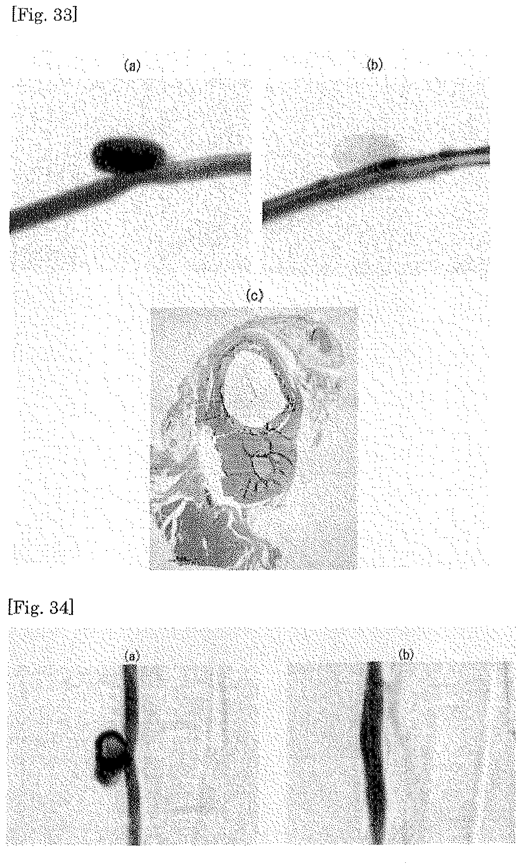

Figure 34(a) is a radiograph of the medical device before indwelling, and

Figure 34(b) is a radiograph of the medical device, 5 minutes after indwelling.

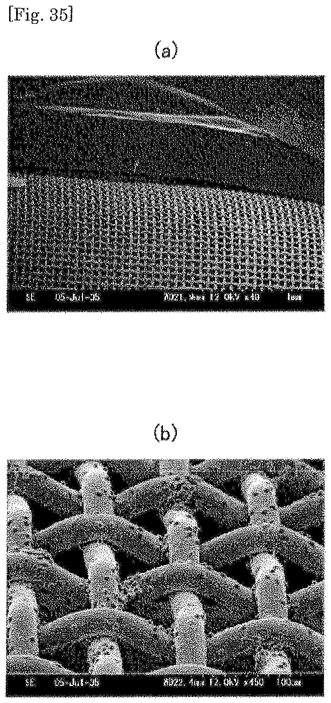

Figure 35 is a SEM photograph of the sample in the aneurysmal phantom-sided region after test in Example 8.

EXPLANATION OF REFERENCES

1:      medical device
2:      main stent body
3:      sheet-shaped member
4:      wire
5:      aneurysm
6:      blood vessel
7:      bonding site
8:      rod
9:      delivery system
10:     protective unit
11:     shaft
12:     hub
13:     second catheter (releasing member)
14:     radiopaque marker
15:     guide wire
16:     catheter
17:     distal end tip
18:     lumen

BEST MODE OF CARRYING OUT THE INVENTION

The medical device for body cavity according to the present invention (hereinafter, referred to as the medical device according to the present invention) is a medical device that can be used for treatment of celomic cavity-related diseases for example, by blocking the openings formed in celomic cavities such as aneurysm, varicosis, and microaneurysm. Particularly in the present invention, the sheet-shaped member has pores and is located as it covers at least part of the main stent body. In such a configuration, the sheet-shaped member extends along the circumferential direction of the main stent body and reduces or blocks blood flow into the opening when the main stent body is expanded, alleviating the load for example to aneurysm, accelerating conversion to thrombus or endothelium in the aneurysm, and blocking the opening safely in a short period of time.

When used in practice, the medical device according to the present invention is first held in the form having the compressed first diameter for facile movement thereof through blood vessel, inserted into the blood vessel, and pushed forward to a lesion close to the vascular wall, in particular to a desired lesion close to the opening in a celomic cavity such as aneurysm, varicosis, or microaneurysm. Subsequently, when the main stent body is expanded, the sheet-shaped member placed in the external or internal surface region of the main stent body moves along the circumferential direction of the main stent body, covering the opening in the celomic cavity with the sheet-shaped member.

Preferably in the present invention, the sheet-shaped member spreads, as it covers at least part of the main stent body, without restricting the movement thereon on the main stent body in the circumferential direction, when the main stent body is expanded, and the shape of the pores of the sheet-shaped member is preserved even after the main stent body is expanded. In the configuration of the present invention, the sheet-shaped member spreads along the circumferential direction of the main stent body when the main stent body is expanded, and thus, in contrast to tubular members that are used in conventional covered stents, the sheet-shaped member does not expand or contract when the main stent body is expanded. Thus, the shape and the structure of the sheet-shaped member itself and also the shape and/or the size of the pores in the sheet-shaped member can be preserved substantially. It is possible in this way to indwell it in the blood vessel reliably with the size of the pores as designed. Particularly in the present invention, as will be obvious from Examples, a mechanism for placement of the sheet-shaped device with the size of the pores as designed is essential, because the size of the pores is important for rapid thrombosis.

Because the sheet-shaped member is spread simultaneously when the main stent body is expanded, the sheet-shaped member and the main stent body are brought into tight contact with each other when the medical device is indwelled in celomic cavity, allowing reduction of the hindrance of blood flow by the resistance of the sheet-shaped member terminal. It can also reduce undesired thrombosis between the sheet-shaped member and the main stent body.

In particular, the sheet-shaped member is preferably made of a mesh, for acceleration of conversion to endodermis and also for flexible fitting thereof to the shape of blood vessel. The mesh is preferably made of a filament material. When used, such a sheet-shaped member made of a filament material, which has a surface area larger than that in a simple mesh structure, allows deposition of platelet thrombi and others on the surface and accelerates conversion to endodermis of the opening region distinctively.

Thus, the medical device according to the present invention in such a configuration is preferably a medical device, comprising

a main stent body formed in a tubular structure and expandable from a compressed first diameter to a second diameter, and

a sheet-shaped member having pores and covering at least part of the main stent body, wherein

the sheet-shaped member spreads as it covers at least part of the main stent body without restricting the movement thereon on the main stent body in the circumferential direction when the main stent body is expanded and the shape of the pores of the sheet-shaped member is preserved even after the main stent body is expanded.

The medical device according to the present invention is more preferably a medical device, comprising a main stent body formed in a tubular structure and expandable in the circumferential direction from a compressed first diameter to a second diameter and a fine-structured sheet-shaped member having multiple pores, wherein the sheet-shaped member spreads, as it covers at least part of the main stent body, together with the expansion of the main stent body in the circumferential direction when the main stent body is expanded and the fine structure of the sheet-shaped member is preserved without change even after the main stent body is expanded.

Hereinafter, each unit of the medical device according to the present invention will be described more in detail.

(Fixation of sheet-shaped member)

The sheet-shaped member according to the present invention is preferably fixed to a region of the main stent body, more preferably in the state in which the substantial preservation of the shape and size of the pores in the sheet-shaped member is not impaired when the main stent body is expanded, for the reasons described above.

The substantial preservation of the shape and size of the pores in the sheet-shaped member means that the rate of the pores (pore rate) is preserved substantially without change when the sheet-shaped member is spread along with expansion of the main stent body.

Also in the present invention, the spread of the sheet-shaped member along with the expansion of the main stent body in the circumferential direction when the main stent body is expanded is advantageous, because the main stent body and the sheet-shaped member are brought into contact with the vascular wall more tightly after expansion, thus preventing problems such as crinkling.

For prevention of uneven expansion of the medical device for body cavity, the sheet-shaped member is preferably fixed so as to cover at least part of the main stent body without restricting the movement on the main stent body in the circumferential direction during expansion of the main stent body.

The fixing site of the sheet-shaped member is arbitrary, if it is a site where the sheet-shaped member can cover the surface of the main stent body, and may be anywhere on the external or internal surface of the tubular-structured main stent body. Fixation of the sheet-shaped member to the main stent body in this way prevents separation of the sheet-shaped member from the stent when the medical device according to the present invention is pushed forward through a lumen structure.

For example if the sheet-shaped member is fixed to the external surface of the main stent body, it is possible to control spread of the sheet-shaped member by the diameter of the stent and also to suppress movement of the sheet-shaped member back around the main stent body after indwelling the medical device.

On the other hand, if the sheet-shaped member is fixed to the internal surface of the main stent body, the sheet-shaped member preferably has a memorized diameter larger than the internal diameter of the stent after expansion, so that the sheet-shaped member fits to the internal wall of the main stent body spontaneously.

Because the medical device according to the present invention has a structure in which a sheet-shaped member is placed on the surface of a main stent body, a sheet-shaped member having a desired length in the circumferential direction may be used as the sheet-shaped member, independently of the length of the main stent body before expansion in the circumferential direction. For that reason, the sheet-shaped member may be selected, for example, according to the opening in the organs having various lumen structures (celomic cavities) or the disorder such as parietal separation. It is also possible to cope with a wider range of disorders, by modifying the length of the sheet-shaped member itself in the circumferential direction.

For example if the sheet-shaped member is placed externally on the main stent body, it is possible to use a sheet-shaped member having a desired length in the circumferential direction without restriction by the length of the external surface of the main stent body before expansion, if the first terminal of the sheet-shaped member edge and the second terminal sheet-shaped member edge are placed, as one of them overlapping part of the other before stent expansion.

In addition, if the sheet-shaped member is placed externally on the main stent body, and in particular if the length of the sheet-shaped member in the circumferential direction is not twice larger than the length of the circumference of the main stent body before expansion, it is desirable to fix the sheet-shaped member at the center in the circumferential direction, to reduce the force needed for spread of the sheet-shaped member for easier expansion and to make it easier to place the sheet-shaped member on the opening of the aneurysm in blood vessel when the sheet-shaped member does not cover the entire circumference. If the length of the used sheet-shaped member in the circumferential direction is larger than twice the length of circumference of the main stent body before expansion, because it is not possible to fold the member without bending of one terminal if it is fixed at the center of the sheet-shaped member in the circumferential direction, it is preferable to fix it at the position closer to one of the terminals (then, if the side of the sheet-shaped member having a shorter length from the fixing site to the terminal, it is preferable to keep the length thereof within the length of one external circumference of the main stent body before expansion).

Also in the present invention, the sheet-shaped member is preferably connected to the main stent body region so that the size of the pores of the sheet-shaped member is preserved substantially during expansion of the main stent body. When it is connected in this way, the size of the pores is kept unexpanded, differently from the case where it is connected entirely onto the surface of the main stent body or to multiple points of the main stent body different in the circumferential direction as in conventional stents, and thus, it is possible to place a sheet-shaped member with the pores as designed.

Especially when a self-expanding main stent body is used, for reduction of the resistance during delivery of the main stent body from the delivery system, the fixing site of the main stent body and the sheet-shaped member is preferably placed at sites on the distal side, preferably more distal than the distal end of the first catheter constituting the delivery system. In particular if the fixing site formed is more distal than the distal end of the first catheter, it is possible to place the sheet-shaped member after the step of placing a main stent body on the first catheter and finally fix it, and thus, it is possible to prepare a delivery system easily. Even when it is fixed to a site more distal from the distal end of the first catheter, if the fixing site is closer to the proximal side, the region extending out of the main stent body expands readily, leading to easier expansion of the distal end and thus, decrease for example in penetrability, and therefore, the fixing site is preferably located in the more distal region.

The fixing site of the main stent body and the sheet-shaped member may be formed internally or externally as it is projected.

Multiple point fixing is more preferable than single point fixing, and linear fixing is furthermore preferable from the point of strength, but the multiple fixing points or the linear fixing part is preferably placed on a line in parallel with the axial direction of the medical device for body cavity, for making the size of the pores of the sheet-shaped member unchanged during expansion of the medical device for body cavity. However, if the flexibility of the distal region of the medical device for body cavity and the delivery system is more emphasized, two-point fixing is more desirable than linear fixing.

(Location of sheet-shaped member on main stent body)

When a self-expanding main stent body is used, the main stent body is preferably made longer than the sheet-shaped member for more reliable spread of the sheet-shaped member (because the expansion force of the main stent body region extending out is transmitted readily to the sheet-shaped member). Such a location is also advantageous in that, even when the sheet-shaped member is not spread sufficiently in the area close to the lesion, the main stent body extending out, which plays a role of anchor by expansion, can prevent movement of the medical device for body cavity by blood flow. In such a case, the sheet-shaped member is preferably placed at a place corresponding to one terminal of the main stent body, for more precise location of the sheet-shaped member to a desired lesion.

(Partial coverage with sheet-shaped member)

If the sheet-shaped member covers the blood vessel entirely on the circumference when the sheet-shaped member is spread, there is no need for particular caution, but, if it does not cover the circumference completely, the sheet-shaped member is preferably indwelled at a position blocking the opening of aneurysm.

The sheet-shaped member and the main stent body can be fixed to each other, by means of a wire rod of metal or resin (e.g., wire, etc.), an adhesive agent, or the like.

If the sheet-shaped member and the main stent body are materials that adhere to each other easily, they may be bonded directly to each other. The bonding may be performed, for example, by inserting the stent terminal directly into the sheet-shaped member. Direct welding, for example with laser, is also possible, but if the damage for example on thin sheet-shaped member and also the influence on the strength of the sheet-shaped member are considered, fixing by other methods is desirable.

(Fixation of sheet-shaped member with wire)

If the sheet-shaped member and the main stent body are fixed with a wire in the present invention, they can be fixed in the movable state, advantageously eliminating the bending load to the medical device for body cavity, the load during stent expansion, and the force applied to the fixing point during production. If a wire is used, the sheet-shaped member may have an opening for introduction of the wire.

For example if the sheet-shaped member and the main stent body are fixed by means of a wire, they may be sewn with the wire, or they may be fixed by means of introducing the wire for bonding into the holes formed on them. For reliable fixation, the sheet-shaped member or the main stent body may be bonded to the wire with laser (for example, YAG laser), adhesive agent, or the like. However, if laser is used, the strength of the sheet-shaped member and the main stent body may decline by heating, and thus, caution should be given when laser is used. On the other hand, it is also possible to form a medical device in which the sheet-shaped member and the main stent body are tightly bound to each other by using a polymeric adhesive agent. When the wire is made of a polymeric material, the bonding substance may be more compatible with it than with a metallic bonding material, if it is a polymeric adhesive agent. Because it is possible to form a strong bonding by fixation by enclosing the wire and the stent strut with a polymeric adhesive agent, fixation by means of a polymer adhesive agent can also be used favorably. The polymer adhesive agent is not particularly limited, if it is a biocompatible adhesive.

In a very small-sized medical device such as the medical device for body cavity according to the present invention (e.g., medical device used for the purpose of blocking the opening of aneurysm), fixation by sewing with a wire has been considered difficult, from the technological point of view. However, after intensive studies, the inventors have developed a new sewing method of forming a seam by feeding a wire having an angle formed by bending in the distal region (the angle of the wire distal region may be formed previously or by operation of a rod, as described below) from the external surface of a main stent body and/or a sheet-shaped member to the internal surface and additionally, feeding the distal end of the wire once again outward by pushing a rod having a diameter similar to the internal diameter of the medical device into the lumen, and thus, succeeded in producing a medical device in such a configuration. The seam may not be formed by feeding a wire both through the main stent body and the sheet-shaped member, and may be formed by feeing the wire only through a region of one member, or alternatively by operation in combination thereof.

The angle in the wire distal region is not particularly limited, but preferably about 10 to 90°. The length of the distal region is preferably larger than the thickness of the main stent body, or if needed larger than the total thickness of the sheet-shaped member and the main stent body.

A rod having a diameter close to the internal diameter of the main stent body is preferably inserted into the internal surface side of the main stent body for support during sewing, for the purpose of preventing damage on the shape of the main stent body. The degree of insertion of the rod into the main stent body is not particularly limited, if the passage of wire is unhindered. The diameter of the main stent body may be reduced then. The rod used then may be hollow or dense. For example, tweezers may be used similarly, replacing the rod.

A hole allowing passage of the wire may be formed previously in the sheet-shaped member. In this case, the sheet-shaped member may be sewn for example with tweezers, as it is placed on the main stent body.

When the sheet-shaped member and the main stent body are to be sewn with a wire (as described above, preferably on a line in parallel with the axial direction of the tubular article), it is possible to fix them, for example, by fixing the wire to a main stent body only by two points and feeding the wire through the sheet-shaped member at more points, and it is possible in this way to raise the flexibility of the medical device for body cavity and also prevent uneven expansion during expansion, because deformation of the strut of the main stent body normally accompanying the expansion is restricted by the wire.

The terminal of the wire used for sewing the sheet-shaped member and/or the main stent body (however, the position is not necessarily limited to the terminal) is preferably fixed to the sheet-shaped member or the main stent body, for example by means of fusion, adhesion (e.g., with a polymeric adhesive agent) or the like. In this case, the wire is preferably welded or adhered to the main stent body, particularly from the point of easiness in processing. When the medical device according to the present invention is expanded to the second diameter, for reduction of the hazard of the site, which is fixed for example by means of fusion or adhesion (which often has protrusions), damaging the blood vessel, the fixing site onto which the wire is fixed is preferably formed on the side not in direct contact with the blood vessel, i.e., the internal surface side by fusion or adhesion (however, when the object of the wire fixed for example by means of welding or adhesion is a region having both the sheet-shaped member and the main stent body, and particularly when the wire is fixed as it is placed on the internal surface, the fixing site may be formed on the external surface side of the object placed inside).

Methods of fixing the sheet-shaped member and the main stent body include various methods such as welding and adhesion, but, particularly as described above, a fixing method by sewing is desirable, because it allows fixation in the movable state, and the spotwise fixing at multiple points often gives improved fixing strength.

When the main stent body and/or sheet-shaped member are metal materials, the wire material to be used is, for example, a polymeric material or a metal material. In particular, a wire of the metal identical with that of the main stent body or the sheet-shaped member is preferably used, from the points of biological compatibility and strength and also for the purpose of preventing the corrosion due to galvanic electrode formation. Examples of the polymeric materials include polymer adhesive agents and the like.

When the sheet-shaped member and the main stent body are fixed to each other by sewing with a wire, coarse sewing with the wire (at a wide interval between bonding regions) results in flexible fixing. Alternatively, dense sewing with the wire (small interval between bonding regions) results in strong fixing action. As for the fixing site, the sheet-shaped member can be fixed from both surfaces, top and bottom surfaces.

A medical device not containing any substances other than the sheet-shaped member and the main stent body and wire (for example, polymer adhesive agent) can be prepared by sewing. In contrast to the bonding by using a polymer adhesive agent, the bonding in this way is desirable, because the fixing is not influenced by the compatibility between the polymeric material and the material for the sheet-shaped member or main stent body.

(Fixation of sheet-shaped member and main stent body with ring)

The sheet-shaped member and the main stent body may be fixed with a ring, and the spotwise fixing similar to that by adhesion can be performed by using a ring but not using an adhesive. It is possible to form each fixing region isolated from each other in contrast to the method of using a wire, and thus, the method is desirable, because the flexibility is less reduced than the bonding fixing with a wire. Further, it would be possible to obtain a strength effect similar to that obtained by sewing, by fixing them by using multiple rings.

The cross-sectional shape of the ring is arbitrary, and may be circular, polygonal (e.g., square) or the like. Examples of the materials for the ring include metal materials and polymeric materials. The material for the ring is preferably the metal identical with that for the main stent body or the sheet-shaped member, similarly to the material for the wire.

If the polymeric material for the ring is biodegradable, it is possible to prepare a medical device that is highly strong and flexible (characteristics of the ring) during placement but has a lower content of foreign materials to the body (by decomposition of the ring) after indwelled.

(X-ray-impermeable marker)

An X-ray-impermeable material is preferably contained in the sheet-shaped member, for rapid delivery of the medical device according to the present invention to a site close to the targeted aneurysm and accurate coverage of the opening with the sheet-shaped member. It is possible in such a configuration to determine, by radiography, the direction of the sheet-shaped member spread.

If an X-ray-impermeable marker is contained in the fixing site on the straight line in parallel with the axial direction of the main stent body, especially when the fixing site is located in the center of the sheet-shaped member, it is possible to locate the center of the sheet-shaped member and the main stent body in the area close to the center of the opening of aneurysm and thus to block the opening easily and reliably.

It is also possible to determine by radiography the position and spread of the sheet-shaped member, by using a sheet-shaped member having an X-ray-impermeable filament material at least partially. Even in such a case, it is preferable in particular to make the visibility of the central X-ray-impermeable material different from that of other regions, for easier confirmation of the position of the center of the sheet-shaped member by radiography. It is also preferable to prepare an X-ray-impermeable material in a design allowing differentiation of radiographs observed from the front and rear faces of the device. The X-ray-impermeable material may be contained in the main stent body.

In the present invention, a biocompatible metal such as SUS, Co-Cr or Ni-Ti can be used favorably as the metal material for the sheet-shaped member, main stent body and wire. On the other hand, the sheet-shaped member, the main stent body and the wire can be prepared with various polymers, and the polymers are preferably polymeric materials compatible to each other (for example, combination of readily fusing polymers or combination of polymers that are bonded to each other with a favorable adhesive agent available), more preferably biocompatible polymeric materials.

(Biocompatible polymer)

Examples of the biocompatible polymers include polyvinylalcohol (PVA), cyanoacrylates, biodegradable materials, glycolic polylactate acid copolymers (PLGA) and the like. For example, a sheet-shaped member and a main stent body may be bonded with PVA or sewn with a filament material of a PVA polymeric material. Alternatively, the sheet-shaped member and the main stent body can be bonded to each other directly with a cyanoacrylate adhesive. Independently of whether the materials are bonded or sewn, the sheet-shaped member can be fixed onto the main stent body spotwise, linearly. When the polymeric material is biodegradable, it is decomposed or eliminated after blockage of the aneurysmal opening, for example, after conversion of the surrounding sheet-shaped member into endodermis. Although polylactic acids are often used as the biodegradable polymeric materials, PLGAs, copolymers thereof with glycolic acid, are favorably used for control of decomposition speed and biocompatibility.

(Area of sheet-shaped member)

In the medical device according to the present invention, if the length of the sheet-shaped member is larger than the circumferential length of the first-diameter main stent body, the sheet-shaped member is configured to make the first terminal of the sheet-shaped member edge overlapped with the second terminal of the sheet-shaped member edge.

For example, during contraction of the main stent body, the sheet-shaped member has a structure in which one terminal of the sheet-shaped member has the other terminal of the sheet-shaped member folded inside. In this case, the terminal of the internal sheet-shaped member can be fed to the fixing site between the main stent body and the sheet-shaped member. The sheet-shaped member folded inside does not cover the main stent body in an angular range of 360° from the fixing site or in an area of more than 100% of the entire surface of the main stent body (stent area). Accordingly, when the terminals of the sheet-shaped member are superimposed on the stent circumference in an angular range of 340° or less or in a stent area of 95% or less, i.e., in the area where the region in which coverage and expansion thereof is difficult is excluded from the stent circumference in an angular range of 360° or the stent area of 100%, it is possible to prepare a medical device that is readily expandable (expansion in the radial direction of the main stent body in the superimposed region above is not inhibited, and thus, restriction on spread of the region folded there inside is prevented) and allows preservation of the area of the covering sheet-shaped member wider during indwelling. As described above, the medical device according to the present invention having a configuration in which a sheet-shaped member is wound on the main stent body surface allows insertion even into a fine blood vessel, as the entire diameter thereof is reduced for insertion during contraction, and in particular, the first diameter of the main stent body is preferably smaller for use of the medical device for body cavity according to the present invention. It is possible to spread the sheet-shaped member extensively during expansion, allowing adaptation to the openings of celomic cavities in various sizes. The coverage angle is the maximum angle covering the main stent body, as seen on the cross section of the sheet-shaped member.

The region of the sheet-shaped member folded inside is preferably smaller, because the expansion force from inside is transmitted to the external sheet-shaped member more effectively. Therefore, the terminal folded inside preferably covers the circumference of the first-diameter stent in an angular range of 60° or more and 300° or less.

It is also possible to reduce the diameter of the medical device for body cavity or the delivery system according to the present invention, by making the area of the sheet-shaped member smaller in consideration of the size of the opening for example of aneurysm. It also leads to improvement in flexibility of the entire device. Reduction in device diameter and improvement in flexibility are effective in feeding the medical device according to the present invention through a fine convoluted blood vessel.

It is possible by selecting the shape of the sheet-shaped member properly to control the area of the main stent body covered by the sheet-shaped member and the amounts of the materials for the entire device (e.g., metal content). The metal content in the sheet-shaped member or the filament material is preferably a value stimulating in-vivo conversion to endodermis of 16.3 mg/mm$^2$ or less, as shown in Example 1.

(Selection of the region covered by sheet-shaped member)

It is effective to reduce the area of the sheet-shaped member for improvement in flexibility of the medical device. However, if the area of the sheet-shaped member is restricted to a particular value, the rate of the area covering the blood vessel declines, as the diameter of the blood vessel applied becomes larger, possible prohibiting sufficient coverage of the lesion by the area of the sheet-shaped member. Reduction in the area of the sheet-shaped member for improvement in flexibility is reversely correlated with the area covering the lesion, and thus, it is important to select the cover region carefully according to its application.

Table 1 shows the relationship among stent external diameter, coverage angle and cover length.

[Table 1]

| Relationship among stent external diameter, coverage angle and cover length | | | | | | | | |
|---|---|---|---|---|---|---|---|---|
| Before expansion | External diameter of main stent body[mm] | External circumference of main stent body [mm] | | | | | | |
| | 1.5 | 4.71 | | | | | | |
| After expansion | | | Coverage angle and covering length by mesh [mm] | | | | | |
| | External diameter of main stent body [mm] | External circumference of main stent body [mm] | 20° | 60 ° | 120 ° | 180 ° | 240 ° | 300 ° |
| | 2 | 6.28 | 0.35 | 1.05 | 2.09 | 3.14 | 4.19 | 5.24[1] |
| | 3 | 9.42 | 0.52 | 1.57 | 3.14 | 4.71[1] | 6.28[1] | 7.85[1] |
| | 3.5 | 11.00 | 0.61 | 1.83 | 3.67 | 5.50[1] | 7,33[1] | 9.16[1] |
| | 5 | 15.71 | 0.87 | 2.62 | 5.24[1] | 7.85[1] | 10.47[1] | 13.09[1] |
| | 10 | 31.42 | 1.75 | 5.24[1] | 10.47[1] | 15.71[1] | 20.94[1] | 26.18[1] |
| 1): The external circumference of the main stent body after expansion is a length equivalent to or larger than the external circumference of the main stent body before expansion. | | | | | | | | |

For example, in the case of a main stent body having a first diameter of 1.5 mm before expansion, the stent circumference is approximately 4.7 mm. When the main stent body is expanded to a second diameter of 3 mm, the half circumference of the stent is approximately 4.7 mm. In the case of a medical device for body cavity according to the present invention containing a main stent body having an external diameter, i.e., a first diameter before expansion, of 1.5 mm and a sheet-shaped member covering the same circumferentially once without superposition at the first and second terminals, indwelling the medical device for body cavity according to the present invention in a 3-mm-diameter blood vessel results in coverage in an angular range of approximately 180° in the internal surface of the blood vessel. However, indwelling a similar device in a 2-mm-diameter blood vessel results in coverage of the vascular wall in an angular range of 240° or more, because the circumference of the 2-mm-diameter blood vessel is approximately 4.19 mm.

Alternatively if the stent external diameter is 10 mm when the main stent body is expanded, the length of the stent in the circumferential direction needed for coverage of the main stent body by the sheet-shaped member in an angular range of approximately 300° is approximately 26 mm.

If the length of the sheet-shaped member that can wrap the main stent body is 8 mm or less, the sheet-shaped member covers the main stent body in a angular range of approximately 180°, when the second diameter of the main stent body when expanded is 5 mm. Yet alternatively if the external diameter of the main stent body when expanded is 3.5 mm, the sheet-shaped member covers the main stent body in an angular range of approximately 240°. If the stent external diameter of the main stent body when expanded is 3 mm, the sheet-shaped member covers the main stent body in an angular range of approximately 300°.

If the length of the sheet-shaped member in the stent circumferential direction is 5 mm and the second diameter of the main stent body when expanded is 10 mm, the sheet-shaped member covers in an angular range of approximately 60°. If the second diameter of the main stent body when expanded is 5 mm, the sheet-shaped member covers in an angular range of approximately 120°. If the second diameter of the main stent body when expanded is 3.5 mm, the sheet-shaped member covers in an angular range of approximately 180°. If the second diameter of the main stent body when expanded is 2 mm, the sheet-shaped member covers in an angular range of approximately 300°.

If the length of the sheet-shaped member in the stent circumferential direction is 3 mm and the second diameter of the main stent body when expanded is 3 mm, the sheet-shaped member covers in an angular range of approximately 120°. If the second diameter of the main stent body when expanded is 2 mm, the sheet-shaped member covers in an angular range of approximately 180°.

(Case of coverage of first-diameter stent circumference in an angular range of 180 to 340° or a stent coverage area of 50 to 100%)

If the sheet-shaped member overlaps in an amount of a stent circumference in an angular range of 180 to 340° or a stent area of 50 to 100% before stent expansion, the sheet-shaped member may not be spread sufficiently even when the stent diameter is expanded sufficiently, depending on the shape of blood vessel, and the sheet-shaped member may remain covering the main stent body consistently in an angular range of 360°. The medical device may still show treatment action, even if it is indwelled in the affected region as it is not spread sufficiently.

For that reason, the sheet-shaped member preferably has an area larger than that needed for covering the lesion. The sheet-shaped member is preferably overlapped in an amount of 2/3 of the stent circumference, i.e., an angular range of 240° or less or a stent area of 67% or less. The stent external surface coverage angle and the covering area of the sheet-shaped member do not necessarily agree, depending on the shape of the sheet-shaped member. For example, square and triangular sheet-shaped members having the same basal length are different in angle and area, and the areas are different, even if the members have the same covering maximum angle. A medical device suitable to the shape of the sheet-shaped member or the targeted disease (e.g., aneurysm, varicosis or microaneurysm) is desirably used.

(Case of coverage of first-diameter stent circumference in an angular range of 180° or less or a stent coverage area of 50% or less)

If the terminals overlap excessively, the frictional resistance between the terminals during expansion may lead to delayed spread of the sheet-shaped member. If the fixing site is formed in the center of the sheet-shaped member and the terminals of the sheet-shaped member are superimposed in an amount up to an angular range of approximately 180° or a stent area of 50% or less when the stent circumference is contracted, half of the sheet-shaped member does not cover the main stent body, the delivery system is thinned in diameter, and both terminals are pushed outward in the same direction when the main stent body is expanded. It is spread allowing reduction in the frictional resistance between two parts of the sheet-shaped member. In addition, flexibility is an important factor in delivery of the medical device in blood vessel, and reduction in diameter and in the superimposed area between the terminals of the sheet-shaped member leads to softening of the medical device for body cavity according to the present invention and also to softening of the delivery system. Increase in flexibility of the delivery system allows supply of the system to a lesion present in a bent or fine blood vessel. For example in the case of blockage of the opening of unruptured aneurysm, it is possible to cope with it by blocking half of the blood vessel diameter, and

a delivery system containing a sheet-shaped member not having a coverage area larger than half of the diameter of the blood vessel may be chosen, while its flexibility is emphasized.

If, in a sheet-shaped member having the first and second terminals of the sheet-shaped member superimposed, these two terminals overlap each other in an area of a stent circumference in an angular range of 180° or less or a stent area of 50% or less during contraction of the main stent body, the sheet-shaped member may be too large, depending on the size of the opening in celomic cavity, the diameter and the shape of the blood vessel. If the targeted blood vessel is a fine blood vessel, the superposition of the terminals may become undesirably excessive eventually, compared to the opening, and the sheet-shaped member may not be spread rapidly by the frictional resistance between the terminal faces during spread. Such a phenomenon may occur particularly frequently in the case of a highly bent blood vessel. The covering area may be the minimal needed, in the case of a tight-necked unruptured aneurysm. Thus by making a sheet-shaped member having the first and second terminals of the sheet-shaped member when the stent circumference is contracted have a configuration in which the terminals overlap on the stent circumference in an angular range of 60° or less or a stent area of 17% or less, it is possible to prepare a medical device higher in flexibility, while leaving most of the thickness of the tubular sheet-shaped member to be that of the single layer.

(Case of coverage of first-diameter stent circumference at an angle of 40° or a stent coverage area of 12%)

If the sheet-shaped member is superimposed on the stent circumference at an angle 40° or a stent area of 12%, it is possible to prepare a flexible medical device containing a smaller amount of the sheet-shaped member, a foreign material to the body.

If the medical device according to the present invention is placed in an area close to the opening of a celomic cavity, the center of the sheet-shaped member should be adjusted to be close to the central region of the opening. The medical device is preferably rotatable and adjustable in the circumferential direction then. For rotation in the circumferential direction, the maximum diameter of the delivery system is preferably 50% or less of the diameter of the targeted celomic cavity.

(Diameter of expanded main stent body)

The first diameter of the main stent body in the present invention means the minimum diameter when the main stent body is compressed. The first diameter is arbitrary, if it is a diameter allowing insertion of the main stent body in a patient's blood vessel such as artery, and varies according to the kind of blood vessel, but it is preferably 5 mm or less, and the internal diameter of the main stent body is preferably 0.01 to 0.04 inch (0.25 to 1.02 mm).

The second diameter of the main stent body is the diameter when the main stent body is expanded (expanded stent diameter). When the expanded stent diameter is 10 mm or less, the medical device can be installed into fine blood vessel to large blood vessel. The main stent body is preferably expanded uniformly under uniform application of a radial force. Therefore, favorable is a main stent body expanded properly and uniformly according to the diameter of the blood vessel where it is indwelled.

The main stent body, if expanded from a first diameter to a second diameter, is preferably expanded in a straight-tube form or in a tapered-tube form. The main stent body preferably has a shape compatible with the shape of the blood vessel.

The medical device according to the present invention can possibly be used in practice in circumstances where partial coverage is difficult or in emergent circumstances such as unexpectedly ruptured aneurysm. In such a case, important is swift coverage of the targeted blood vessel. Preferably for easier and swifter operation, the entire circumference of the main stent body is covered with the sheet-shaped member during expansion. The "entire circumference" includes the case where the sheet-shaped member covers the stent circumference once or more (in an angular range of not less than 360°). Examples thereof include one circumference, approximately 1.5 circumferences, approximately 2 circumferences, and the like.

If an aneurysm is formed in a blood vessel system suitable for expansion of the main stent body in a lesion where coverage of the entire circumference of the blood vessel is difficult, because the sheet-shaped member covers the stent circumference in an angular range of up to 300° or a stent area of up to 84% after expansion, it is possible to indwell the medical device easily even at the site where the opening of the celomic cavity is very wide. Even if the position of the sheet-shaped member in medical device is dislocated in the circumferential direction during expansion of the stent or because of problems in operation, because the sheet-shaped member covers the stent circumference in an angular range of up to 300° or a stent area of up to 84% after expansion, it is possible to obtain an area sufficiently covering the blood vessel for example with aneurysm. In addition, even if the blood vessel diameter is larger than expected, it has an area sufficient for coverage in a similar manner. The medical device according to the present invention is advantageous in that it can cover the opening of aneurysm safely, even if the aneurysm is different in its form and opening area.

To reduce the amount of the sheet-shaped member, a material foreign to the body, and yet to have an area of the indwelled sheet-shaped member sufficient for coverage, the sheet-shaped member preferably covers the stent circumference in an angular range of up to 240° or a stent area of up to 67% after expansion. It is favorable as a spreadable sheet-shaped member even when the vascular wall opposite to the lesion has branches or when the main stent body is expanded only slightly or insufficiently.

Among aneurysms formed in blood vessel systems suitable for expansion of the main stent body, there are some aneurysm cases wherein the sheet-shaped member need not cover the stent circumference in an angular range of up to 240° or a stent area of up to 67% after expansion. Thus for reduction in the amount of the used sheet-shaped member, a material foreign to the body, and for sufficient preservation of the area of the indwelled sheet-shaped member, the sheet-shaped member preferably has a coverage after expansion of a stent circumference in an angular range of up to 180° or a stent area of up to 50%. If the sheet-shaped member is rectangular, when the external diameter is 1.5 mm during stent contraction and the expansion diameter of the main stent body 3.0 mm, the coverage angle of the sheet-shaped member on the stent is 180° after expansion. This is equivalent to the sheet-shaped member covering the entire circumferential of the main stent body during contraction of the main stent body.

It is possible to block a wide-neck unruptured aneurysm, by covering the area corresponding to half of the blood vessel diameter.

Among aneurysms formed in a blood vessel system suitable for expansion of the main stent body, there are some aneurysm cases wherein the sheet-shaped member need not cover the stent circumference in an angular range of up to 180° or a stent area of up to 50% after expansion. Thus for reduction in the amount of the sheet-shaped member, a material foreign to the body, and for sufficient preservation of the area of the indwelled sheet-shaped member, the sheet-shaped member preferably has a coverage of the stent circumference in an angular range of up to 120° or a stent area of up to 34% after expansion.

A coverage of the stent circumference in an angular range of less than 180° after expansion leads to improvement in flexibility of the medical device having the stent after expansion. It can be indwelled in an aneurysm at a bent site.

A configuration in which the sheet-shaped member covers the stent circumference in an angular range of up to 60° or a stent area of up to 17% reduces the amount of the used sheet-shaped member, a material foreign to the body, and improves the flexibility of the medical device. It is favorable in the case of a tight-necked unruptured aneurysm occurring in a bent blood vessel in which the amount of the covered area needed is smaller.

(Shape of sheet-shaped member)

If the sheet-shaped member is oval or polygonal in shape, the terminal of the sheet-shaped member not essential for coverage of aneurysm can be removed and also, undesired part of the sheet-shaped member, a material foreign to the body, can also be reduced. In particular, an oval sheet-shaped member has an area smaller than the rectangular sheet-shaped member. In addition, reduction in area of the sheet-shaped member leads to improvement in flexibility of the medical device for body cavity according to the present invention. It may be a polygonal sheet-shaped member.

On the other hand, in the case of a 4-point rectangular sheet-shaped member, even if the medical device is dislocated in the axial direction, it is possible to use it completely to the terminal for coverage of the aneurysmal opening and to make it show an action similar to that when the medical device is indwelled with the central region of the sheet-shaped member facing the center of the aneurysmal opening.

For indwelling in a large opening of celomic cavity or at a site of a blood vessel where the diameter thereof is large, a rectangle model with four vertexes is desirable for assurance of sufficiently large coverage area.

When the medical device according to the present invention is indwelled, the curvature memorized by the outermost circumference of the sheet-shaped member is preferably larger than the curvature of the external circumference of the main stent body at the second diameter, because such a medical device prevents folding part of the sheet-shaped member into the opening of the main stent body during expansion of the main stent body and makes it expanded more easily. It is also preferable when the sheet-shaped member contacts the blood vessel internal wall earlier during indwelling medical device and the sheet-shaped member is subsequently held down by the main stent body, because it decreases friction between terminals of the sheet-shaped member and the main stent body and prevents breakdown of the sheet-shaped member caused by forced spread of the sheet-shaped member.

In addition, a folded or wavy structure of the sheet-shaped member, which makes it spread easily in the direction opposite to the main stent body side, is more preferable for prevention of the folding lines of the sheet-shaped member into the main stent body or for earlier spread of the sheet-shaped member.

For example, at least part of the sheet-shaped member is preferably processed to have folding lines at an angle of 0° to 179° in the direction different from that in the main stent body side. In addition, the sheet-shaped member is preferably processed to memorize the wavy shape.

Even for a sheet-shaped member, for example of SUS, that does not have shape memory function, it is preferable to make it have a curvature greater than that of the first-diameter stent, folding, or wavy structure for automatic spread of the sheet-shaped member, thereby making it have a function equivalent to the shape memory function for spread of the sheet-shaped member without resistance.

If the sheet-shaped member has nicks at least one site, the nicks reduce bending resistance and improve flexibility of the main stent body during contraction. It is also advantageous for delivery of the medical device through a bent channel.

The method of forming the nicks, the number and the location thereof, and others are not particularly limited, if the sheet-shaped member is stable enough against breakdown by expansion of the main stent body. The degree of nicking is not particularly limited, but, for example, the nick preferably extends in the region from the terminal to the fixing site of the sheet-shaped member folded into the innermost side or in the region from the terminal to the central region from the viewpoint of easiness in folding the sheet-shaped member.

(Structure of sheet-shaped member and pores)

As for the thickness of the sheet-shaped member, the thickness of the terminal is smaller than that of the center of the sheet-shaped member, which leads to reduction in thickness when the terminals are superimposed and thus prevents reduction in flexibility due to increase in thickness. The change in partial thickness and partial structure of the sheet-shaped member, the fluctuation in strength by the size of the pores of the sheet-shaped member and the number of the pores are factors in designing the flexibility of the sheet-shaped member.

The pores are formed, as they penetrate the sheet-shaped member from one face to the other. It is possible to control the flexibility of the sheet-shaped member in the axial and diameter directions arbitrarily, for example, by making the pores of the sheet-shaped member have a shape uneven in directional size such as oval or rectangular. Thus for improvement in flexibility of the medical device during contraction or for improvement in flexibility of the sheet-shaped member after expansion of the medical device, the shape of the pores of the sheet-shaped member or the arrangement thereof may be modified.

(Arrangement of the pores of sheet-shaped member)

Even arrangement of the pores of the sheet-shaped member gives a sheet-shaped member uniformity in flexibility. Alternatively, uneven arrangement of the pores of the sheet-shaped member gives a sheet-shaped member locally different in flexibility. Presence of high-density pores leads to easier blood flow (through the sheet-shaped member), while presence of low-density pores to hindered blood flow. It is possible to adjust the blood flow into the aneurysmal opening with the positional pattern of the pores. It is possible to control the blood flow into the aneurysm for example by lowering the pore density for reducing the blood flow into aneurysmal opening and raising the pore density for increasing the blood flow to the celomic cavity side. It is possible in this way to control the blood flow into and out of aneurysm and the blood pressure and thus to reduce the risk of aneurysmal rupture after indwelling. In addition, the region of the sheet-shaped member higher in pore density is higher in flexibility.

(Surface shape of sheet-shaped member)

Both surfaces of the sheet-shaped member are preferably very smooth, for reduction of the surface friction of the sheet-shaped member. It is possible in this way to reduce the damage of the medical device for body cavity according to the present invention, by friction to the vascular wall when inflated and also the spread inhibition and breakdown of the medical device by friction of the overlapping parts of the sheet-shaped member during expansion of the medical device.

It is also possible to increase the friction of the sheet-shaped member locally, by forming surface irregularity on at least one region in the overlapping region of the sheet-shaped member. The surface irregularity can also prevent unintended spread, as it acts on the face of the sheet-shaped member when the sheet-shaped member is stored folded, while the medical device is contracted.

The surface irregular structure also increases the surface area of the sheet-shaped member. The increase in surface area is advantageous, because it accelerates deposition of biological substances such as platelet and induces earlier thrombosis of the pore in the sheet-shaped member and earlier blockage of the opening for example of aneurysm. The surface irregular structure may be the wrinkling of the sheet-shaped member formed when the medical device is contracted to the first diameter.

The surface irregular structure evenly formed on the sheet-shaped member increases the surface area of the sheet-shaped member. If the sheet-shaped member has surface irregularity in the terminal region, the friction of the sheet-shaped member is increased in the terminal region. Thus when the sheet-shaped member is stored as enfolded during contraction of the medical device, unintended spread of the sheet-shaped member, even with overlapping terminals, can be prevented. The surface irregular structure can be formed on surface, for example by embossing a planar sheet-shaped member. It may be wrinkling. The height of the raised structure (in the thickness direction of the sheet-shaped member) is preferably 0.05 to 0.15 mm, and the difference between the surfaces of the raised structure and the dent structure is preferably 0.05 to 0.55 mm.

(Thickness of sheet-shaped member)

It is possible to change the average thickness and the strength of sheet-shaped member slightly, by modifying the degree of the surface irregularity of sheet-shaped member. The surface area and the flexibility of the sheet-shaped member may be altered, by combination of the surface irregularity different in degree and the distribution density thereof.

A sheet-shaped member having an average thickness of 0.2 mm or less gives a medical device containing a sheet-shaped member having a large size of pore or a pore rate and a strength preserved. The size of pore is the diameter of the pore when it is circular, the maximum length of the vertical line from an apex to the facing base when it is triangular, the maximum length of the base when it is quadrangular, or the length of the longest diagonal line when it is polygonal, and the pore rate is the rate of the pore area in a certain area of the sheet-shaped member.

When the average thickness of the sheet-shaped member is 0.08 mm or less, it is possible to prepare a medical device improved in flexibility, by reducing the size of pore or the pore rate of the sheet-shaped member. Such a sheet-shaped member gives a medical device remaining flexibility when contracted and remaining strength of the sheet-shaped member after spread. As shown in Example 1, a medical device containing a sheet-shaped member having an average thickness of 0.08 mm or less is a medical device allowing long-term growth of vascular endodermis on the sheet-shaped member after indwelling.

A medical device containing a sheet-shaped member having an average thickness of 0.05 mm or less is a medical device further improved in flexibility. It is a medical device that remains more flexible when contracted and strong of the sheet-shaped member when expanded. As shown in Examples 2, 3 and 6, a medical device containing a sheet-shaped member having an average thickness of 0.05 mm or less is a medical device allowing medium or short-term growth of vascular endodermis on sheet-shaped member after indwelling.

Further as shown in Examples 4, 5 and 7, a medical device containing a sheet-shaped member having an average thickness of 0.03 mm or less is a medical device more flexible when contracted.

Adjustment of the thickness of the sheet-shaped member leads to softening of at least one region of the sheet-shaped member, and the difference in thickness due to overlap of the sheet-shaped member exerts an influence on the flexibility of the medical device when contracted. It is possible to prepare a delivery system that can be delivered through a bent region, by making the terminal regions of the sheet-shaped member thicker and stronger and reducing the thickness of the internal sheet-shaped member for improvement in flexibility.

(Strength and flexibility of network-like structure)

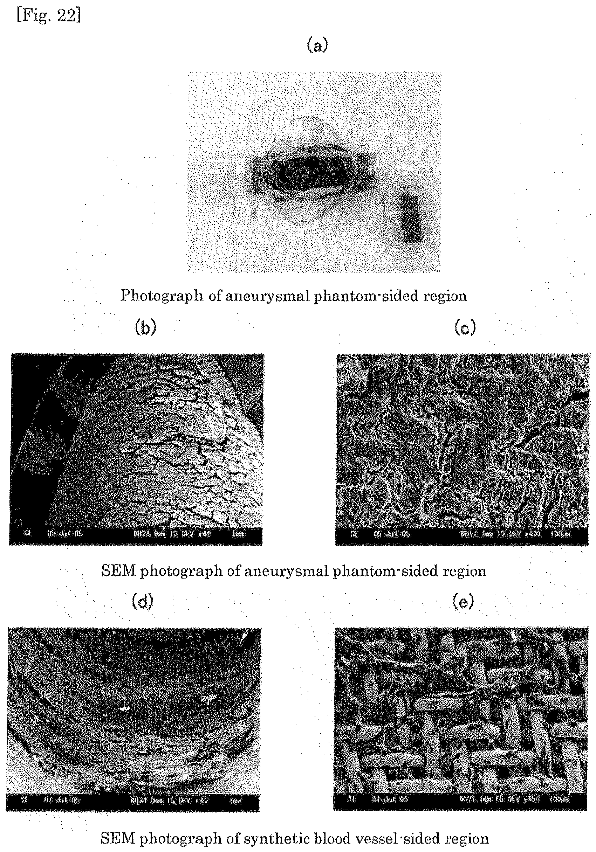

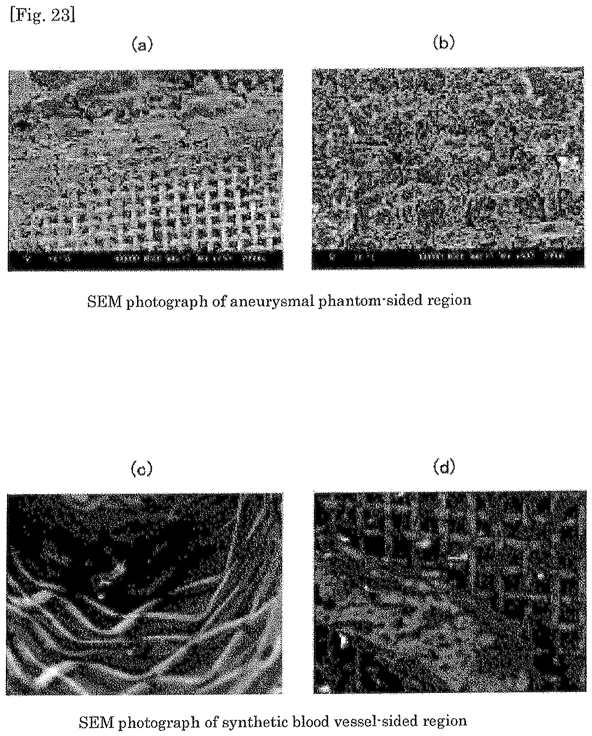

When the sheet-shaped member is a mesh having a network-like structure, it is possible easily to make the pore rate and the pore diameter uniform. In addition, the strength of the sheet-shaped member can also be made uniform. Further, when the sheet-shaped member has a network structure, the surface area in a unit region is increased. Consequently, it leads to easier deposition of platelets during blood flow through the pores in the network-like structure, acceleration of thrombosis, earlier blockage of the aneurysmal opening, and conversion to endodermis. Coverage of the aneurysmal opening with the sheet-shaped member makes the blood remain in the aneurysm, consequently leading to thrombosis of the aneurysm-sided surface of the sheet-shaped member as shown in Figures 22 and 23 and additionally long-term conversion to endodermis as shown in Figure 33(c). The flexibility of the sheet-shaped member having a network-like structure can be expressed by: density of sheet-shaped member $\times$ (wire diameter or wire thickness/size of pore).

The density of the sheet-shaped member is a filling factor of the sheet-shaped member (raw material) in a certain volume, and is concerned with the resistance by filling. The value (wire diameter or wire thickness/ size of pore) is concerned with the resistance inherent to the sheet-shaped member designed. The flexibility of the sheet-shaped member is estimated to be lower, when the value of the Formula is higher. The mesh sheet-shaped member, which has fine openings for example in a porous or network-like structure, has a mass of sheet-shaped member smaller than that of a flat plate-shaped sheet or a porous flat plate-shaped sheet and a smaller adhesion area in the blood vessel. In addition, the sheet-shaped member of mesh, which contains pores larger than those in the wire region, gives a medical device more flexible in the bending direction than that in the punching sheet-shaped structure that is prepared by punching a flat plate-shaped sheet.

The sheet-shaped member may have a network-like structure in which a filament material sewn. The network-like structure, which increases the surface area of the sheet-shaped member per unit area, is advantageous for rapid thrombosis.

(Material for sheet-shaped member)

It is also possible to improve the flexibility in at least one region of the sheet-shaped member, by using at least two kinds of different materials or filament materials different in wire diameter in combination. It is advantageous for improvement in flexibility of the medical device during contraction. When the sheet-shaped member has a network-like structure of a metal material, even a sheet-shaped member made of a filament material higher in strength and smaller in filament diameter can retain a certain strength for example against tensile stress. Because metal materials are stronger, there is a reduced risk of breakage of the metal-shaped member because of spread of the metal-shaped member during expansion of the medical device. The wire mesh is preferably made of a metal material with assured biocompatibility.

The diameter of the filament material is preferably smaller than the thickness of the main stent body region, and it is more preferably not larger than the thickness of the stent main bodies used in the Examples of 0.2 mm or less, as shown in the present description. The wire diameter is the average wire diameter of the filament material.

(Network structure of network-structured member)

A sheet-shaped member made of plain woven product is a strong sheet-shaped member uniform in thickness and size of pores. The plain woven article is a mesh characteristically woven alternately with warp and weft filament materials. A sheet-shaped member having such a plain woven structure has a filament material density lower than those obtained by the other weaving method and the distance between the filament materials is larger. Use of a low-density plain-woven sheet-shaped member made of a small-diameter filament material leads to improvement in the flexibility of the delivery system. A special example of the plain woven mesh is plain Dutch weaving.

If the sheet-shaped member is a twill mesh, it is a sheet-shaped member higher in the density of the filament material than the plain woven member. The twilled weave is a weaving method characterised by interlacing each other warp and weft filaments, and gives a flexibility similar to that by plain weave, even if a wire having a larger diameter is used. The twill-woven sheet-shaped member is improved in strength and higher in density than the plain-woven product, has a larger surface area per unit area, and is favorable for acceleration of deposition of biological substances such as platelet. A special example of the twilled weave is twilled Dutch weave.

If the sheet-shaped member is plain Dutch weave mesh, it is a sheet-shaped member having a high mesh number and a high density. The plain Dutch weave is a weaving method of forming a mat-like mesh by using warp filaments and bundles of fine weft filaments in combination, and such a mesh is favorable for application of filtration of fine particles present in fluid. Thus, the high-density sheet-shaped member has a larger surface area per unit area and is favorable for acceleration of deposition of biological substances such as platelet.

If the sheet-shaped member is a twilled Dutch weave mesh, it is a sheet-shaped member having a mesh number further higher than that of plain Dutch weave. The twilled Dutch weave is a weaving method of forming a mat-like product by weaving warp filaments each of which interlaces over several weft filaments and weft filaments and such a mesh gives a sheet-shaped member in which the distance of the filament material is uniform, independently of its filament diameter. The distance of the filament material has an influence on the size of the pores of the sheet-shaped member and also on deposition of the biological substances such as platelet. It is thus advantageous for equation of the size of the pores width of the sheet-shaped member produced in combination of filament materials.

(Shape of filament member)

When the sheet-shaped member has a mesh made of a filament material in the wavy shape, it has a woven-type network-like structure. It is also possible to change the thickness of the sheet-shaped member partially or the frictional resistance partially, by modifying the amplitude of the waveform for example by weaving.

If the sheet-shaped member is made of a spirally-shaped filament material, it becomes a sheet-shaped member having a network-like structure different from those prepared by normal weaving methods. It is also possible to change the thickness of the sheet-shaped member partially or the frictional resistance partially, by modifying the internal or external diameter of the spiral. Such a spiral shape may be formed as an anchor at the terminal of the filament material. If the filament material having at least two spiral shapes at the center of the sheet-shaped member is used, it is possible to use the spiral shapes as part of fixing sites, by feeding the wire for fixation of the sheet-shaped member to the stent into the spiral therein.

It is possible to obtain a sheet-shaped member locally flexible, by using at least two kinds of weaving methods in combination. Even if two kinds of weaving methods are used, it is possible to obtain a sheet-shaped member with two or more regions different in flexibility, for example, by using filament materials having wavy shapes different in the amplitude of waveform.

Even if the filament material in the sheet-shaped member has a wire diameter of 0.2 mm or less, during expansion of the medical device containing a sheet-shaped member larger in size of pore or pore rate, the strength of the sheet-shaped member is preserved after spread. The wire diameter is the average of the wire diameters of respective filament materials.

If the filament material in the sheet-shaped member has a wire diameter of 0.04 mm or less, the medical device become improved in flexibility, if the size of pore or the pore rate is reduced. It gives a medical device remaining flexible when contracted, in which the strength of the sheet-shaped member is preserved after spread. As shown in Example 1, the medical device containing a sheet-shaped member, in which the filament member has a diameter of 0.04 mm or less, becomes a medical device allowing long-term growth of vascular endodermis on the sheet-shaped member after indwelling.

A filament material in the sheet-shaped member having a filament diameter of 0.03 mm or less gives a medical device further improved in flexibility. It also gives a medical device improved in flexibility, relative to its metal content, if the filament member is twill weave. As shown in Example 2, the medical device, of which the filament member in the sheet-shaped member has a diameter of 0.03 mm or less, becomes a medical device allowing middle-term growth of vascular endodermis on the sheet-shaped member after indwelling.

If the filament material in the sheet-shaped member has a filament diameter of 0.02 mm or less, it gives a medical device higher in flexibility. As shown in Examples 3 and 6, a medical device containing a filament member in sheet-shaped member having a diameter of 0.02 mm or less becomes a medical device allowing middle-term growth of vascular endodermis on the sheet-shaped member after indwelling.

Use of at least two kinds of filament materials different in wire diameter in combination gives a sheet-shaped member locally flexible.

The flexibility of the network-structured sheet-shaped member varies according to the wire diameter of the filament member. For example, a sheet-shaped member consisting of a filament material having a larger diameter and a filament material having a smaller diameter in ordered or random combination is more flexible locally or entirely than a sheet-shaped member made of a single filament material.

(Size of pore of sheet-shaped member)

A sheet-shaped member containing pores having a size of pore of 0.2 mm or less that is made of a large-diameter filament member becomes a medical device that can reduce the amount of the used sheet-shaped member, a foreign material to the body during coverage of the celomic cavity opening with the sheet-shaped member. The size of pore can be determined by using Micro-Hi-scope, SEM, TEM, laser analyzer, or the like.

A medical device having pores allows growth of vascular endodermis on the sheet-shaped member after indwelling. When a sheet-shaped member containing pores having a size of pore of 0.1 mm or less is used, a medical device having pores becomes a medical device allowing long-term growth of vascular endodermis on the sheet-shaped member after indwelling, as shown in Example 1,

When the sheet-shaped member containing pores having a size of pore of 0.06 mm or less is used, a medical device having pores becomes a medical device allowing middle-term growth of vascular endodermis on the sheet-shaped member after indwelling, as shown in Example 2.

When a the sheet-shaped member containing pores having a size of pore of 0.03 mm or less is used, a medical device having pores becomes a medical device allowing short-term growth of vascular endodermis on the sheet-shaped member after indwelling, as shown in Examples 3 to 6.

It is also possible to prepare a partially flexible sheet-shaped member, by using at least two kinds of filament materials different in size of pore width in combination. The flexibility of the network-structured sheet-shaped member varies according to the size of pore of the filament member. Large-size of pore weaving method and small-filament diameter weaving method in ordered or random combination gives a sheet-shaped member locally or entirely more flexible than sheet-shaped members of a single configuration.

(Pore rate of sheet-shaped member)

A medical device containing a sheet-shaped member having a pore rate of 80% or less leaves a smaller amount of the sheet-shaped member as foreign material during coverage of the celomic cavity opening with the sheet-shaped member. Because it is a medical device having pores, it becomes a medical device for body cavity according to the present invention that allows easier growth of vascular endodermis after indwelling. A medical device for body cavity according to the present invention containing a sheet-shaped member having pores rate of 50% or less allows middle- or long-term growth of vascular endodermis on the sheet-shaped member after indwelling. The pore rate can be expressed by:

$$(\text{size of pore}/(\text{size of pore} + \text{wire diameter}))^2 \times 100, \text{ or } (\text{space area }/\text{entire}$$

$$\text{surface area}) \times 100.$$

Each parameter can be calculated, based on at least one test data.

(Main stent body)

The main stent body for use in the present invention may be a stent expandable from the state contracted for example under pressure, and may be, for example, a stent used in PTCA technology or in cerebrovascular operation.

An example of the main stent body is a stent formed in the tubular structure. The length of the tubular article in the longitudinal direction is preferably 3 to 25 mm, for production of a sheet-shaped member suitable for a particular celomic cavity opening. The medical device for body cavity according to the present invention is required to be indwelled also in a lesion of bent blood vessel, and thus, the main stent body is preferably highly flexible in the contracted state.

In the tubular main stent body, the tubular article has a lumen penetrating therein in the axial direction for insertion of a deployment device such as balloon catheter, and the main stent body has various design patterns engraved on the side wall area according to the expansion pattern. The internal diameter of the tubular article, and the marked pattern thereon are not particularly limited, and may be determined properly for example according to the fixing mode of the sheet-shaped member, targeted lesion, and the diameter of the blood vessel used.

Particularly in the case of the self-expanding medical device according to the present invention, if only parts of the main stent body and the sheet-shaped member are fixed to each other, preferably proper designs are engraved on respective parts. Examples of the designs include ring- and hole-like structures for introduction of the filament material that are used when the main stent body and the sheet-shaped member are fixed to each other as they are sewn with a metal or PVA filament material.

(Expansion pattern of main stent body)

The main stent body may be a type of product that is expanded by a device such as balloon or a self-expandable type product. When placed at the targeted position, the main stent body in the medical device is expanded to spread the sheet-shaped member, and consequently the medical device according to the present invention should cover the celomic cavity.

If the main stent body is not expanded favorably by resistance of the sheet-shaped member, the main stent body may be expanded additionally to the optimal diameter by using a post-expansion balloon catheter. Thus, the sheet-shaped member may be still in the folded state immediately after indwelling.

(Material and properties of main stent body)

The material for the main stent body is preferably a metal, for prevention of breakdown during spread of the sheet-shaped member and for support for spread of the sheet-shaped member. If the material for the sheet-shaped member is also a metal, the sheet-shaped member is resistant to breakdown during spread and folding back into the blood vessel after spread and covers the aneurysmal opening consistently.

The metal material is preferably SUS, SUS302, SUS304, SUS316, or SUS316L, more preferably SUS or SUS316, because it gives a medical device superior in corrosion resistance, mechanical strength and biocompatibility. As shown in each Example, SUS316L is a material especially corrosion resistant among the SUS products, and it gives a biocompatible medical device superior in corrosion resistance to non-oxidative acid, pitting, and corrosive gas.

If the metal material is Co-Cr, it gives a medical device superior in corrosion resistance, mechanical strength and X-ray-impermeability.

Alternatively if the metal material used for the medical device is a self-expandable metal having superelastic action, it gives a medical device that returns to its original shape by removal of load, even if deformed significantly at a temperature slightly higher than the transformation temperature. When the metal material has shape memory effect, if the main stent body has memory of the shape in the high-temperature phase, it returns to its original shape when heated to the body temperature in the body, if it is deformed at a temperature not higher than the martensite transformation temperature. In particular, if the material is Ni-Ti, it gives a biocompatible self-expandable medical device.

It is possible to make a medical device resistant to breakdown during spread of the sheet-shaped member by using the same metal for the main stent body and the sheet-shaped member and to suppress galvanic corrosion caused by difference in ionization tendency particularly by using the same metal for most of the medical device members.

The dimension such as thickness and length of the main stent body is not particularly limited.

(Fixed structure of sheet-shaped member)

It is possible to obtain a medical device with separated sheet-shaped members, by fixing at least two sheet-shaped members onto the periphery of a main stent body. If the sheet-shaped members are separated, it is possible when the medical device is in the first shape to eliminate the bending resistance by the delivery system with the gap and to give a medical device improved in flexibility.

It is possible to prepare stent main bodies different in the second shape, by fixing the sheet-shaped member by using at least two stent main bodies. It is a medical device that can be indwelled in a blood vessel having a smaller diameter or in a branched blood vessel having branches different in blood vessel diameter. It is possible to prepare a medical device locally different in flexibility, by using main stent bodies different in kind for modification of local flexibility.

When the sheet-shaped member is fixed to the center of the main stent body in the major axis direction, it is possible to give a medical device allowing placement of the center of the sheet-shaped member in the axial direction in front of the aneurysmal opening, by locating the center of the medical device on the aneurysm opening. When the sheet-shaped member is not fixed to the terminal side of the main stent body, it is possible to reduce the expansion pressure when the medical device for body cavity according to the present invention is expanded. The expansion pressure needed during conversion to the second shape is lower in a device in which the main stent body and the sheet-shaped member are fixed to each other in the central region than, for example, in a device, in which the terminal of the sheet-shaped member is fixed to the main stent body and is released spirally from one direction. Such a configuration is advantageous for prevention of balloon rupture when the medical device is a balloon-expansion device and for reduction of concern about insufficient expansion when it is a self-expandable device.

(Applications of the inventive medical device)

The medical device for body cavity according to the present invention can block aneurysmal opening rapidly and induce conversion to endodermis in the area where the sheet-shaped member is indwelled. Currently, a protecting device such as protecting balloon or filter device is used for prevention of scattering of thrombotic debris during indwelling of a stent, for example during treatment of carotid artery embolism. However, when thrombotic debris is not removed with a protecting balloon or a filter device, the thrombotic debris flows into the head, possibly clogging the blood vessel there that bears biologically important functions. In contrast, it is possible to confine fine thrombotic debris without scattering, more effectively than with conventional protecting devices, by indwelling the medical device according to the present invention in a lesion of carotid artery embolism. The medical device according to the present invention, which uses as the sheet-shaped member a mesh having pores finer than those in filter device, allows rapid conversion to endodermis.

(Delivery system)

The medical device according to the present invention in the configuration above is delivered into a targeted celomic cavity by various delivery systems. The delivery system delivering the medical device according to the present invention to a lesion can be selected properly according to the expansion type of the medical device, and may be a device with or without a balloon catheter, or possibly a capsule-like device, not a catheter, of which the region holding the medical device is digested in body after placement of the medical device.

For example if the sheet-shaped member and the main stent body of the medical device according to the present invention do not have self-expanding function, it is, for example, a delivery system having the medical device according to the present invention placed on a balloon catheter. The medical device is expanded and spread by inflation of the balloon structure in this case.

If the sheet-shaped member and the main stent body of the medical device according to the present invention have self-expanding function, it is a delivery system consisting of a medical device according to the present invention, a first catheter, and a second catheter that can push out the medical device. It is desirable in this case that the entire medical device according to the present invention or the main stent body thereof is placed in the internal tubular side of the first catheter, and if appropriate, the sheet-shaped member is placed externally around the tubular region of the first catheter, and thus, all or part of the medical device according to the present invention is indwelled in a lesion, as it is separated from the tubular delivery system.

The guide wire used during delivery by the delivery system may pass through the inner side of the first-diameter main stent body or may pass through a guide-wire lumen prepared separately. The catheter for the delivery system may be a monorail-type catheter or an over-the-wire-type catheter.

The monorail-type catheter is a type of catheter delivering only the distal region of a delivery system to the targeted site along a guide wire, and examples thereof include a type of catheter having an opening for guiding a guide wire into an internal cavity that is formed on the side wall close to the distal end of the delivery system, a type of catheter having another tube in the area close to the distal end of the delivery system, a type of catheter having an opening on the side wall in the distal-end tip region, and the like. The over-the-wire-type catheter is a catheter having a lumen for delivery of guide wire, extending to both ends of the delivery system.

(Expansion of balloon-expansion-type medical device)

The balloon unit in the delivery system for supply of the balloon-expansion-type medical device should contain a balloon having a diameter needed for expansion of the medical device according to the present invention. For example when the medical device according to the present invention is to be expanded to 3.5 mm in diameter, desirable is a delivery system containing a balloon having a diameter of 3.5 mm subtracted by the thickness of the sheet-shaped region and the main stent body. If the thickness of the sheet-shaped region and the main stent body is small or for simple and intuitive consideration, the external diameter of the balloon may be 3.5 mm.

(Expansion of self-expanding medical device)

In the case of a delivery system delivering a self-expanding medical device, all or part of the medical device according to the present invention should be installed in the delivery system. The medical device according to the present invention may be placed in one cavity, and preferably, the main stent body of the medical device according to the present invention may be placed in the lumen side and the sheet-shaped member, outside the delivery system or between the external surface of the delivery system and the lumen. The placement of the sheet-shaped member outside the delivery system is performed, for example, by winding the sheet-shaped member around the external surface of the delivery system.

If the sheet-shaped member is placed outside the delivery system, the delivery system is preferably constructed with a protective unit in order to prevent the sheet-shaped member from receiving pressure and from contacting with undesired biological substances on the surface. Protection of the exterior sheet-shaped member in a tube or protective unit is advantageous for prevention of the damage on blood vessel internal wall by the sheet-shaped region during delivery of the medical device according to the present invention through blood vessel and also for prevention of unexpected difficulty in expanding the medical device caused by folding lines and others encountered when the sheet-shaped member is brought into contact with the vascular wall during delivery.

For example when the self-expanding medical device according to the present invention is expanded to 3.5 mm in diameter, a self-expanding stent should be used that is expandable to a diameter of 3.5 mm subtracted by the thickness of the sheet-shaped region and the main stent body. For simple and intuitive consideration, a main stent body having an external diameter of 3.5 mm should be assumed.

(Structure of delivery system for self-expanding medical device)

If at least one of the sheet-shaped member and the main stent body in the medical device according to the present invention has self-expanding function, it is preferable that the entire medical device according to the present invention or the main stent body thereof is placed in the delivery system tubular region and the medical device according to the present invention is indwelled or can be indwelled in the lesion when all or part of the medical device is released from the placement site. It is more preferable that at least one region of the sheet-shaped member is placed in the protective unit outside the tubular region and the medical device according to the present invention is indwelled or can be indwelled in the lesion when all or part of the medical device is released from the placement site. Particularly when the sheet-shaped member and the main stent body are fixed to each other, respectively via the distal points or regions around them, it is preferable that the sheet-shaped member excluding the fixing site is placed in the protective unit side and the main stent body are stored in the internal tubular side, and the medical device is indwelled or can be indwelled in the lesion when the medical device is released from the placement site of the medical device.

(External diameter of delivery system for self-expanding medical device)

The maximum external diameter of the delivery system for delivery of the medical device according to the present invention is preferably made smaller for smooth delivery thereof in blood vessel. The maximum external diameter of the distal-end thereof is preferably 2.5 mm or less when the delivery system is indwelled in a carotid artery lesion, and the maximum external diameter is particularly preferably 2.0 mm or less when it is indwelled in a cranial lesion.

(Method of releasing self-expanding medical device by pushing)

The self-expanding medical device in the present invention can be indwelled, when the medical device or the main stent body placed in the delivery system is pushed forward. If the sheet-shaped member is fixed to the main stent body, the sheet-shaped member is preferably placed in blood vessel, as it is pushed together with the main stent body simultaneously. Preferably when the medical device is released from the delivery system, a second catheter is used for release. The second catheter may or may not be hollow. The material for it may be a polymer, a metal, or a combination thereof. A guide wire may extend in the lumen of hollow second catheter, and the internal lumen may be used for suction of foreign materials, thrombotic debris and others. In particular, the second catheter is preferably a tube having an internal diameter of 0.01 to 0.04 inch (0.254 to 1.016 mm).

(Method of releasing the self-expanding medical device by pulling the cover region)

The self-expanding medical device according to the present invention can be indwelled by removing cover region by the delivery system and the protective unit. The cover region is more preferably removed by pulling the region toward the proximal side. If the sheet-shaped member is covered with the protective unit outside the delivery system, desired is a structure in which the delivery system is connected to the protective unit and, when the delivery system is pulled, the protective unit is also withdrawn simultaneously, and thus the main stent body and the sheet-shaped member are released simultaneously. For release of the self-expanding medical device as it is separated from the first catheter, desired is a structure in which the main stent body is fixed in such a manner that the main stent body does not move with the delivery system during separation of the delivery system.

(Method of releasing self-expanding medical device independently)

For release of the self-expanding medical device according to the present invention, if it is a delivery system by which the main stent body is finally indwelled in the lesion as it is covered with the sheet-shaped member, the main stent body and the sheet-shaped member need not be released simultaneously and thus, may be released respectively by independent mechanisms. Preferably in such a case, the main stent body is released after release of the sheet-shaped member. The sheet-shaped member may be released after release of the main stent body, if possible.

If the main stent body and the sheet-shaped member are released independently, each of them need not necessarily be released by pushing. Specifically, the main stent body may be released by pushing, while the sheet-shaped member may be released by other method. If each member is released independently by different methods, the shaft region of the delivery system need not necessarily be tubular. For example, it may be partially not hollow, and also may be partially not tubular, because the delivery system has a different mechanical configuration for example for the reasons of independent release.

Other favorable examples of the delivery system according to the present invention include the followings.

(Advantageous effects of embodiments)

The method of treating an opening formed in celomic cavity (such as aneurysm, varicosis, or microaneurysm) by using the medical device according to the present invention is not particularly limited, if it is a method based on known procedure. For example, the medical device according to the present invention is delivered into the blood vessel leading to the aneurysm, and the main stent body is expanded at the targeted position by using an expanding device such as balloon catheter, thereby placing the sheet-shaped member in the blood vessel as it is pressed to the internal wall thereof.

It can also be applied to treatment for patients suffering from the diseases of circulatory organs such as those related to patient's aneurysm. For example, a medical device expandable from compressed state to expanded state, in which at least part of the external surface of the main stent body is covered with the sheet-shaped member, is placed in patient's lesion, for example by a delivery system, with the sheet-shaped member in contact with the opening side of aneurysm. It is possible to treat the lesion, by reducing the blood flow from the lesion site and the parent's blood vessel, by expanding the main stent body and allowing the sheet-shaped member to cover the opening such as of aneurysm.

The blood flow into the opening for example of aneurysm is reduced by pressing the sheet-shaped member of the medical device or the main stent body region having the formed sheet-shaped member onto the opening of the celomic cavity. Then, a small amount of blood flow passes through the opening, leading to platelet deposition on the sheet-shaped member and thrombosis of the sheet-shaped member surface.

The pores of the sheet-shaped member are preferably smaller, for improvement in blocking efficiency of the opening and for short-term blockage of the blood flow into the opening. For reduction in the amounts of biologically foreign materials and acceleration of platelet deposition and thrombosis by increase in surface area of the sheet-shaped member, those having a great number of small-size pores are preferable.

When the sheet-shaped member is indwelled on the opening of aneurysm, the blood in the aneurysm remains there, accelerating thrombosis more on the surface of the aneurysm-sided sheet-shaped member than on the parent blood vessel-sided surface. For further acceleration of the thrombosis, a mesh of a filament material, preferably of a filament metal material, is desirably used as the sheet-shaped member expanding the surface area thereof.

For prevention of galvanic corrosion, the main stent body and the sheet-shaped member are preferably made of the same metal.

The sheet-shaped member is placed on at least one region in the periphery (side wall) of the main stent body during expansion, it preferably moves on the stent surface during expansion of the main stent body for reduction of external profile. It may be fixed onto the main stent body peripheral surface by adhesion or welding, and need to be fixed at least only temporarily during delivery. When the sheet-shaped member is spread together on the main stent body peripheral surface, the sheet-shaped member is preferably formed with the fixing unit, such as wire, extending along the central line, for easier location thereof to the site corresponding to the lesion such as aneurysmal opening.

Hereinafter, the present invention will be described in detail with reference to drawings, but it should be understood that the present invention is not limited to these Examples.

EXAMAPLES

(Brief description of medical device)

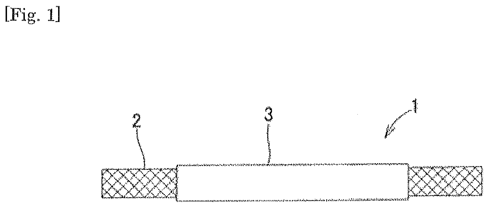

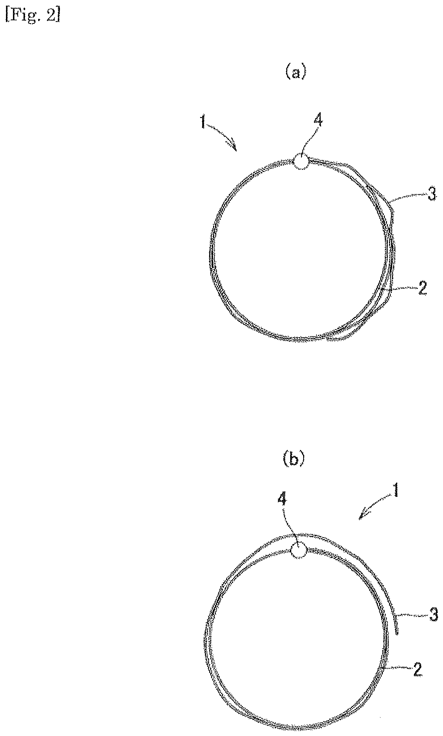

Figures 1 and 2 show a medical device 1 consisting of a main stent body 2 and a sheet-shaped member 3 wound around the main stent body 2. The medical device 1 is delivered by means of a deployment device such as balloon catheter feeding the medical device 1 (not shown in the Figures) in the states shown in Figures 1 and 2.

Figures 2(a) and 2(b) are schematic sectional views showing a main stent body 2 wound with a sheet-shaped member 3 in the circumferential direction. In Figure 2(a), the sheet-shaped member 3 is fixed to the main stent body 2 with a wire 4, and the terminals of the sheet-shaped member are enfolded and overlapped each other in the region to the right side of the wire 4. In Figure 2(b), the sheet-shaped member 3 is wound around the external surface of the main stent body 2 clockwise as seen from the wire 4. It is possible to control the degree of flexibility of the medical device 1 in the circumferential direction, by the winding number of the sheet-shaped member 3 and the strength of the winding. These factors have influences on the flexibility of the deployment device. The deployment device may be, for example, a medical device such as balloon catheter, micro catheter, or guiding catheter.

The main stent body 2 and the sheet-shaped member 3 are expanded from the compressed state of the main stent body 2 by means of a device such as balloon catheter, after release from the fixing device, or under heat.

The present invention is targeted at cases in which the main stent body 2 and the sheet-shaped member 3 are partly fixed to each other, and is not targeted at cases in which the entire surface of the sheet-shaped member 3 is fixed to the main stent body 2 tightly.

The flexibility of the medical device 1 varies, according to the material, structure or design of the main stent body 2 or the sheet-shaped member 3, or the materials for bonding the stent of the main body 2 and sheet-shaped member and the shape of the bonding site.

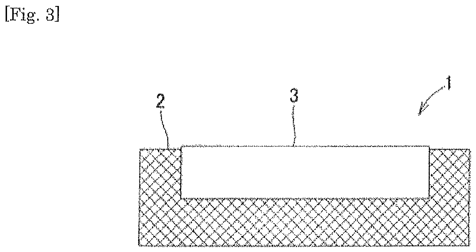

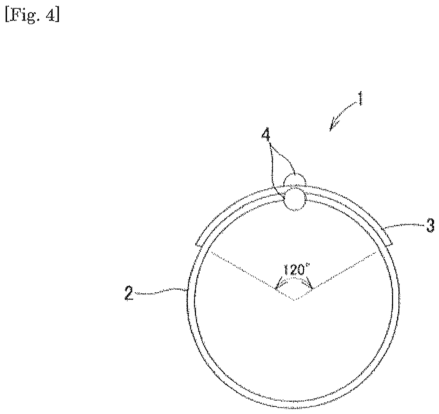

Figures 3 and 4 show a medical device 1 in which the sheet-shaped member 3 is spread around the circumference of the expanded main stent body 2 in an angular range of 120°. Figure 3 is a side view of the medical device 1, while Figure 4 is a schematic sectional view of the medical device 1.

In an embodiment, the sheet-shaped member 3 spreads together with the expansion of the main stent body 2, when the main stent body 2 is inflated with some deformation. In contrast to the expansion of the main stent body 2 with some deformation, the sheet-shaped member 3 shows almost no structural deformation or no change in size of pore and pore rate during spread, and is spread almost independently of the expansion of the main stent body 2.

(Method of introducing medical device)

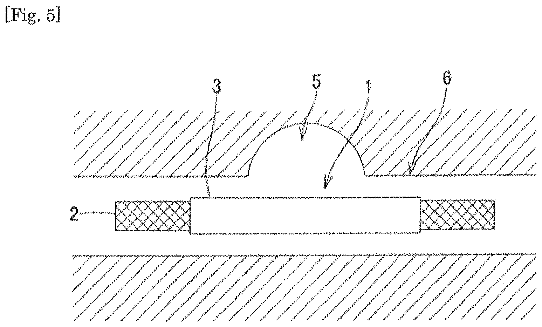

Figure 5 is a view showing the state where a medical device 1 is placed in a region close to the opening of an aneurysm 5, as it is delivered through a blood vessel 6. The step for introduction of the medical device 1 demands a step of preparing a deployment device holding and delivering the sheet-shaped member 3 (not shown in the Figure).

Subsequently, the delivery system is introduced into the blood vessel 6 of the patient.

As shown in Figure 5, the medical device 1 is then located in a targeted region close to the aneurysm 5 formed on blood vessel and the medical device 1 is separated from the deployment device for placement.

Specifically in the case of a balloon-expansion medical device 1, the medical device 1 is placed and fixed on the balloon of a balloon catheter (not shown in the Figure) and delivered to the lesion through a hollow catheter such as guiding catheter. After introduction, the catheter is located accurately for inflation of the medical device 1 at the intended site. The medical device 1 is placed there, as it blocks for example the opening of aneurysm on blood vessel after inflation of the balloon.

In the case of a self-expanding medical device 1, it is placed there in a previously compressed state. The medical device 1 in the compressed state is placed, for example, in a hollow catheter. The catheter is then delivered to the lesion through a hollow catheter and located at the site desirable for inflation of the medical device 1. The medical device 1 is placed and expanded there, for example as it is pushed out of the hollow catheter.

(Inflation of medical device)

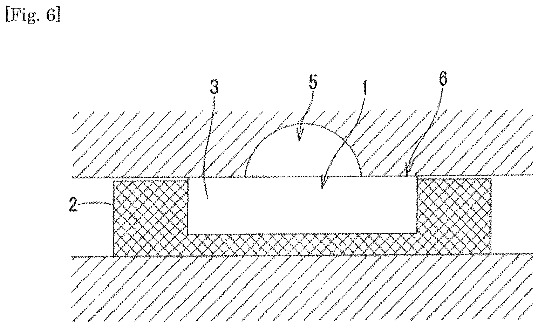

Figure 6 is a view illustrating a state in which the medical device 1 is inflated after placement at the site close to the aneurysm 5, the sheet-shaped member 3 is pressed to the opening of the aneurysm 5, and thus, the blood flow into the aneurysm 5 is reduced or blocked. Shown in Figure 6 is a state of a sheet-shaped member 3 spread (inflated) together with the main stent body 2 because the sheet-shaped member 3 is fixed to the external surface of the main stent body 2. The sheet-shaped member 3 following the external surface of the main stent body 2 is pressed and fixed to the opening of the aneurysm 5 as the main stent body 2 is inflated at the site close to the aneurysm 5.

The sheet-shaped member 3 covers the opening of aneurysm 5 as it follows the main stent body 2, and coverage of the opening of aneurysm 5 with the sheet-shaped member 3 enables reduction and blockage of the blood flow into the aneurysm 5.

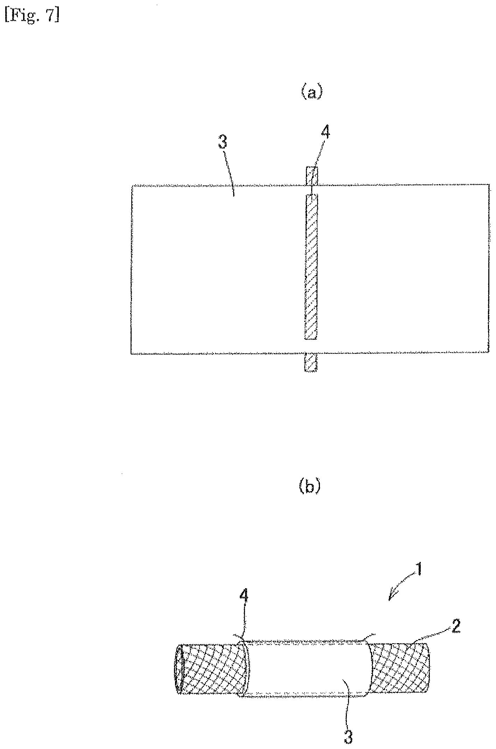

Figures 7(a) and 7(b) are views illustrating a state of the sheet-shaped member 3, as seen from the side where it is fixed to the main stent body (the side in contact with main stent body 2 for example in Figure 1). As shown in Figures 3 and 4, the sheet-shaped member 3 in Figure 7(a) has a wire 4 installed for stabilization of the sheet-shaped member 3 during expansion of the main stent body 2 and during spread thereof along the peripheral surface of the main stent body 2. As shown in Figure 7(b), the sheet-shaped member 3 and the main stent body 2 are fixed to each other with the wire 4 for example by welding or by using a polymer adhesive agent. The areas coated with the adhesive agent are preferably the wire 4 and the internal side of the terminal of stent 2.

The sites of the main stent body 2 and the sheet-shaped member 3 fixed to each other may be in the terminal of the sheet-shaped member 3, but, are desirably located in places other than the terminal, such as the central portion, as shown in Figure 7.

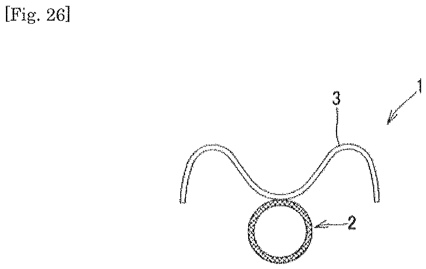

For example, when the sheet-shaped member 3 is fixed in the terminal region as shown in Figure 2(b), the sheet-shaped member 3 spreads by expansion of the main stent body 2 as it slides on the main stent body from inside. If the medical device 1 is inflated then by the balloon catheter or the main stent body 2, it would extend radial outward in the circumferential direction along the major axis of the main stent body 2 at the center. Such a structure demands large pressure for spread of the sheet-shaped member 3. In addition, the wound sheet-shaped member 3 should receive a force mainly directed vertically to the circumference of the main stent body 2 by inflation of the main stent body 2, and the wound sheet-shaped member 3 should be expanded vertically to the circumferential direction of the main stent body 2 and then in the circumferential direction. The internally wound sheet-shaped member 3 should then spread, as it slides between the main stent body 2 and the sheet-shaped member 3 externally covering the same. For spread of the sheet-shaped member 3 without resistance, the sheet-shaped member 3 is preferably wound and contracted around the main stent body, after it is molded into the shape shown in Figure 26.

If the sheet-shaped member is fixed in the non-terminal region as shown in Figure 2(a), the internal and external parts of the sheet-shaped member 3 are spread in the circumferential direction respectively in directions different from each other. Actually when a medical device 1 having a sheet-shaped member 3 of 16x16 mm metal mesh is expanded with a balloon from a diameter of 1.6 mm to 4 mm, the balloon inflation pressure needed for expansion of a sample, in which the main stent body 2 is fixed to the mesh terminal, was approximately 2.75 times larger than that of the sample in which the sheet-shaped member is fixed vertically to a metal mesh, as it is divided into two regions that move in the circumferential direction of the metal mesh at a length rate of 1:2 (4 mm:12 mm). Thus, a medical device having a sheet-shaped member fixed to the non-terminal region of a main stent body, which demands smaller pressure for expansion and thus, smaller load to the balloon, is desirable for prevention of balloon rupture. The wire 4 is desirably present mainly between the main stent body 2 and the sheet-shaped member 3, but for eliminating the disturbance by the wire 4 during expansion of the main stent body 2, the wire 4 is desirably present mainly on the aneurysmal side.

As shown in Figure 7(b), in fixing the sheet-shaped member, the wire 4 is placed in parallel with the axial direction of the main stent body 2, and fixed, preferably sewn, to the surface region of the stent main body 2. There may be only one fixing site, but fixation at least two sites is preferable for tight fixation and smooth expansion of the main stent body 2 and the sheet-shaped member 3.

In addition, the sheet-shaped member 3 and the main stent body 2 can be fixed to each other more tightly with the wire 4 by forming bonding sites additionally.

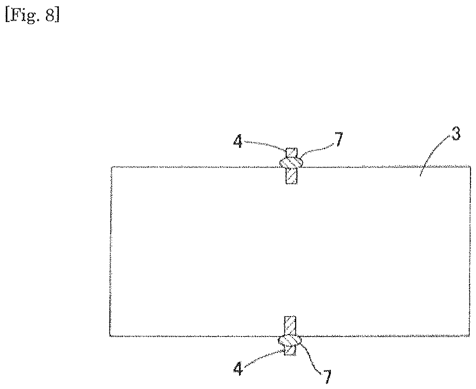

Figure 8 is a schematic view illustrating the side of the sheet-shaped member 3 in contact with aneurysm (side of the sheet-shaped member not in contact with the main stent body), when the sheet-shaped member 3 and the main stent body 2 are fixed to each other at two sites. In the case where there are two bonding sites 7, the bonding sites 7 to the main stent body 2 are preferably present in the region of the wire 4 terminal extending out of the sheet-shaped member 3. Alternatively, at least one of the two bonding sites 7 to the main stent body 2 may be present in the region extending out of the sheet-shaped member 3. Such a configuration is effective for prevention of complete separation of the main stent body 2 and the sheet-shaped member 3.

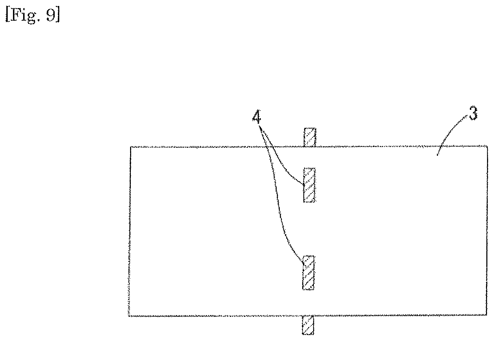

Figures 9, 10, 11 and 12 are schematic views of sheet-shaped members 3 fixed to the main stent body 2 at two, three, and more sites.

In Figure 9, a wire 4 is placed as it penetrates a sheet-shaped member 3 at four sites.

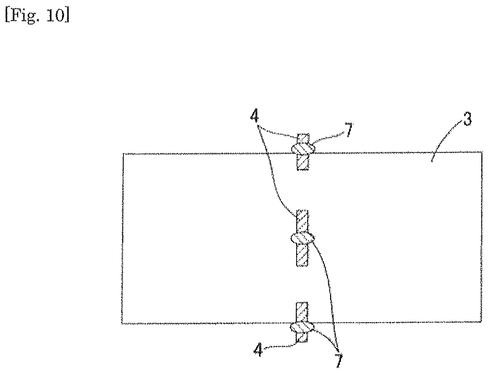

In Figure 10, a wire 4 is placed as it penetrates a sheet-shaped member 3 at four sites and additionally, there are three bonding sites formed.

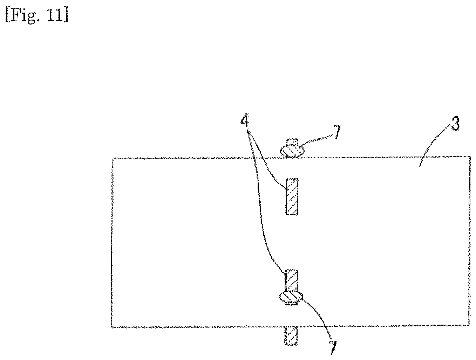

In Figure 11, a wire 4 is placed as it penetrates a sheet-shaped member 3 at four sites and additionally, there are two bonding sites formed.

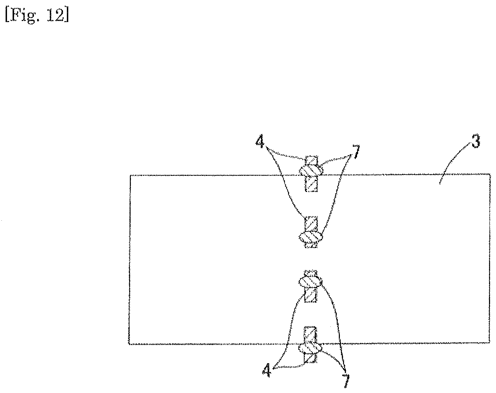

In Figure 12, a wire 4 is placed as it penetrates a sheet-shaped member 3 at six sites and additionally, there are four bonding sites formed.

In Figures 9 to 12, the wire 4 has a structure in which it is sewn to the sheet-shaped member 3 and, in Figures 10 to 12, the bonding sites 7 may or may not be present in the region where the main stent body 2 and the wire 4 are in contact with each other. The wire 4 may be located in any place other than the terminal region of the sheet-shaped member 3. The wire 4 is preferably present mainly between the main stent body 2 and the sheet-shaped member 3, but for eliminating the disturbance by the wire 4 during expansion of the main stent body 2, the wire 4 is desirably present mainly on the aneurysmal side.

As shown in Figures 10 and 12, there may be three bonding sites 7 if the wire 4 appears at three sites on the main stent body 2 and four bonding sites 7 if the wire appears at four sites, and there may be two or more bonding sites 7 on each wire 4 fraction appearing on the main stent body 2 side or no bonding site 7 thereon. The bonding sites 7 include those formed by welding, solder brazing, and also direct sewing with the wire 4 to the main stent body 2.

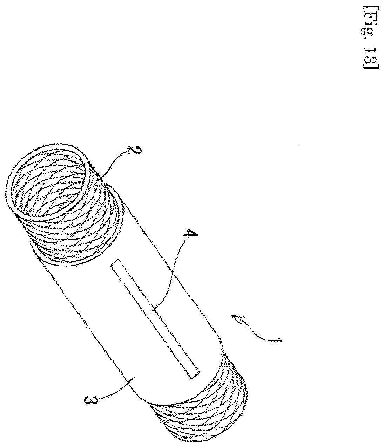

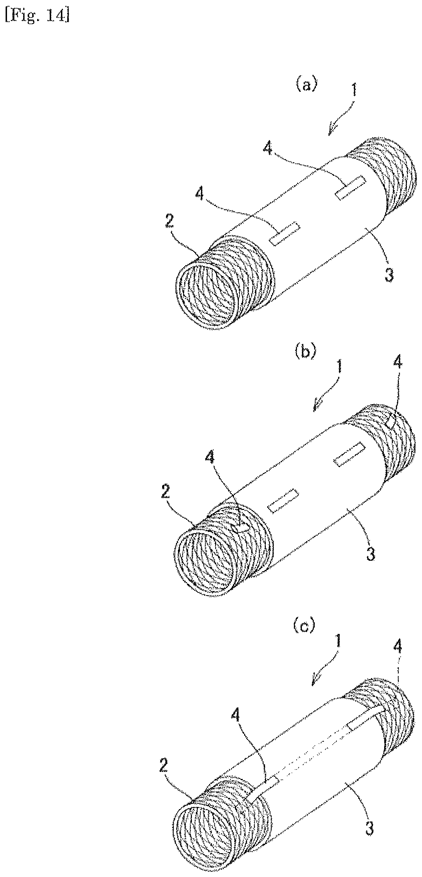

Figures 13 and 14 are schematic views illustrating the shape of the sheet-shaped member 3 before expansion or after expansion when the sheet-shaped member 3 is fixed to the main stent body 2 at two or three sites. In Figure 13, there is the wire 4 located in a greater amount on the aneurysmal side. In Figure 14(a), the wire 4 is present on the aneurysmal side in an amount smaller than that in Figure 13, and in Figure 14(b), the wire 4 is present on the external surface side of the main stent body 2. In Figure 14(c), the wire 4 is present in a shape holding the boundary between the main stent body 2 and the sheet-shaped member 3. The wire 4 may be present in the area between the main stent body 2 and the sheet-shaped member 3 or in the internal side of the main stent body 2.

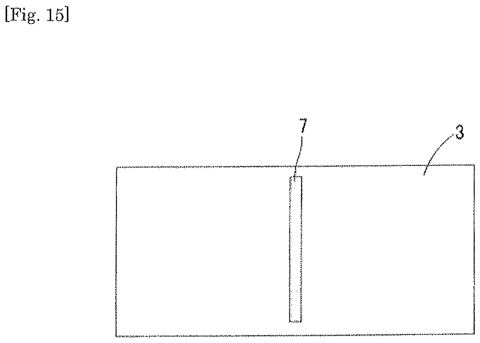

Figure 15 is a schematic view illustrating a sheet-shaped member 3 having a bonding site 7 in the shape of a linear segment. The bonding method may be welding or solder brazing. The length of the bonding site 7 may not be larger than the length of the sheet-shaped member 3 or may be larger for improvement in bonding strength.

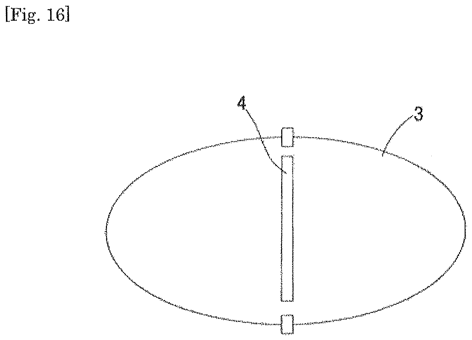

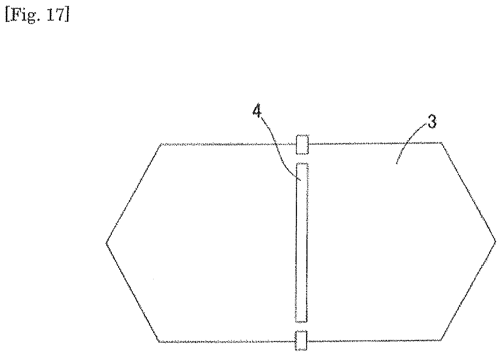

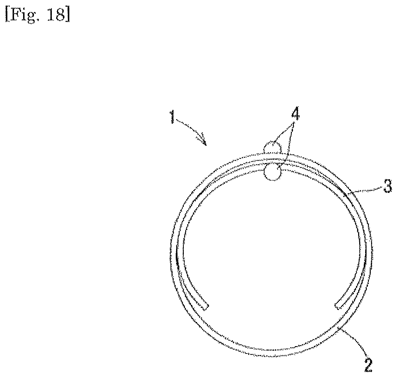

Figures 16 and 17 are schematic views of elliptic and polygonal sheet-shaped members 3. The fixing site may be in the terminal region of the sheet-shaped member 3, but is preferably in the non-terminal region for favorable bonding strength. Figure 18 is a schematic view illustrating a medical device 1 in which the sheet-shaped member 3 is fixed internally to the main stent body 2. In Figure 18, the main stent body 2 and the sheet-shaped member 3 are fixed to each other by using a wire 4, but the main stent body 2 and the sheet-shaped member 3 may be bonded to each other by welding or solder brazing. The main stent body 2 and the sheet-shaped member 3 may be brought into tight contact, by making the former have a curvature larger than the latter.

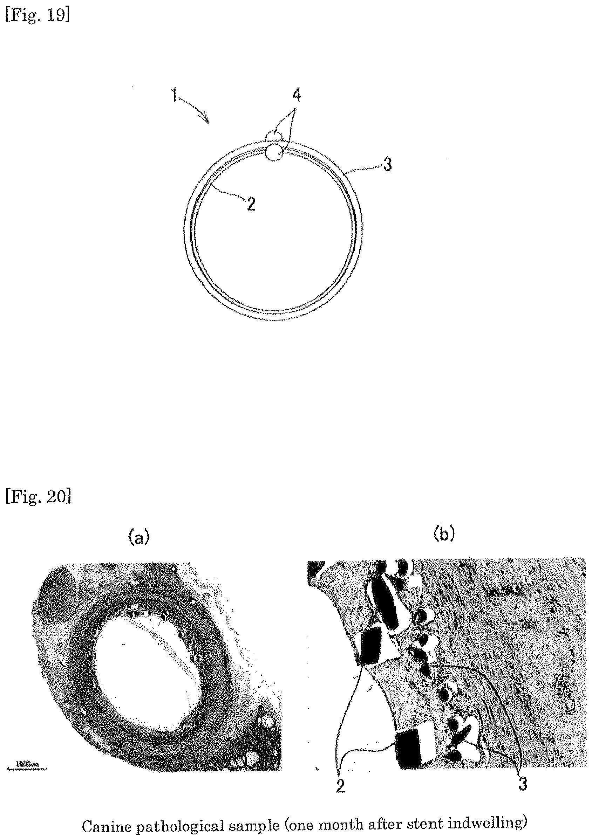

Figure 19 is a view illustrating the state of the medical device 1 in which the sheet-shaped member 3 covers the external surface of the inflated main stent body 2 in an angular range of 360°. In the case of a medical device 1 allowing coverage of the main stent body 2 with the sheet-shaped member 3 in an angular range of 360° after expansion, it is possible to cover the aneurysmal opening, only by placing and expanding the medical device 1 in the area close to the lesion. The direction of expansion of the sheet-shaped member 3 need not be specified during expansion.

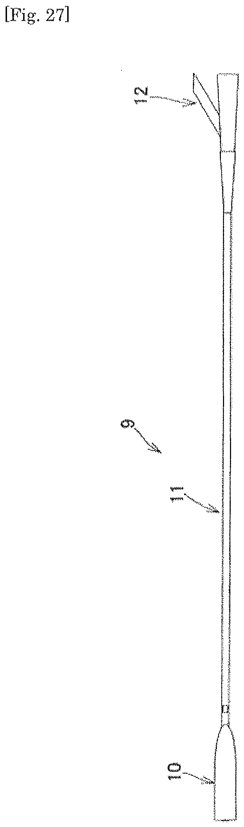

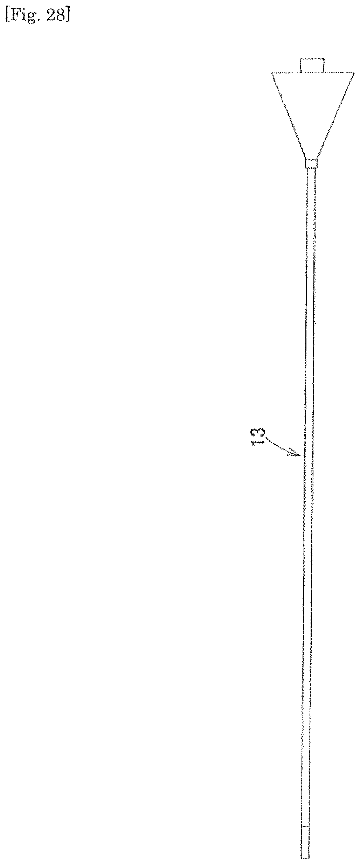

Figure 27 shows a delivery system 9 for self-expanding medical device. It is classified broadly into three regions, a distal-end protective unit 10, a shaft 11 and a hub 12. The medical device 1 is placed in the protective unit 10. The shaft 11 is the shaft of a first catheter. Alternatively, Figure 28 shows a second catheter 13 for releasing the self-expanding medical device 1 from the delivery system 9. The second catheter 13 is inserted from the hub 12 into the lumen of the shaft 11, the medical device 1 is released outward from the delivery system 9, when the proximal-end terminal of the medical device 1 placed in the protective unit 10 is pressed by the distal end of the second catheter 13.

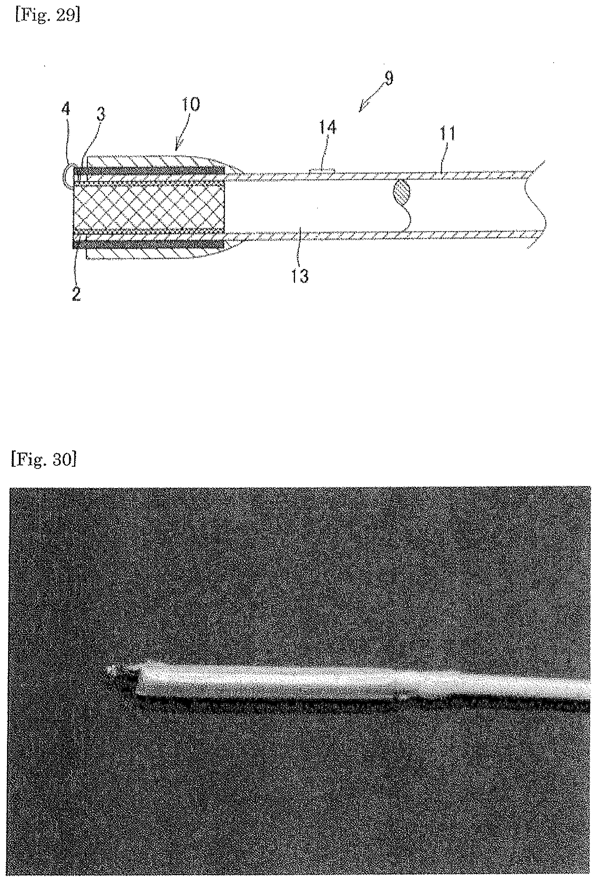

Figure 29 shows the structure of the area close to the protective unit 10 in Figure 27. In the Figure, a main stent body 2 is placed in the lumen of the shaft 11 and a sheet-shaped member 3 between the shaft 11 and the tube constituting the protective unit 10.

Thus, it is a delivery system in which the main stent body 2 is placed in the tube of the delivery system.

The main stent body 2 and the sheet-shaped member 3 are fixed to each other with a wire 4. During indwelling, the self-expanding medical device 1 is released and indwelled, as the main stent body 2 is pushed forward with the second catheter 13 (Figure 28). In the delivery system 9 above, the medical device can be released smoothly, because the main stent body 2 and the sheet-shaped member 3 are placed separately in the distal region of the delivery system 9. It is also possible to determine the position of the delivery system distal region in celomic cavity, by placing a radiopaque marker 14 on the outermost surface of the shaft 11. Figure 30 is a photograph showing the appearance of the distal region of a delivery system prepared in a structure similar to that in Figure 29.

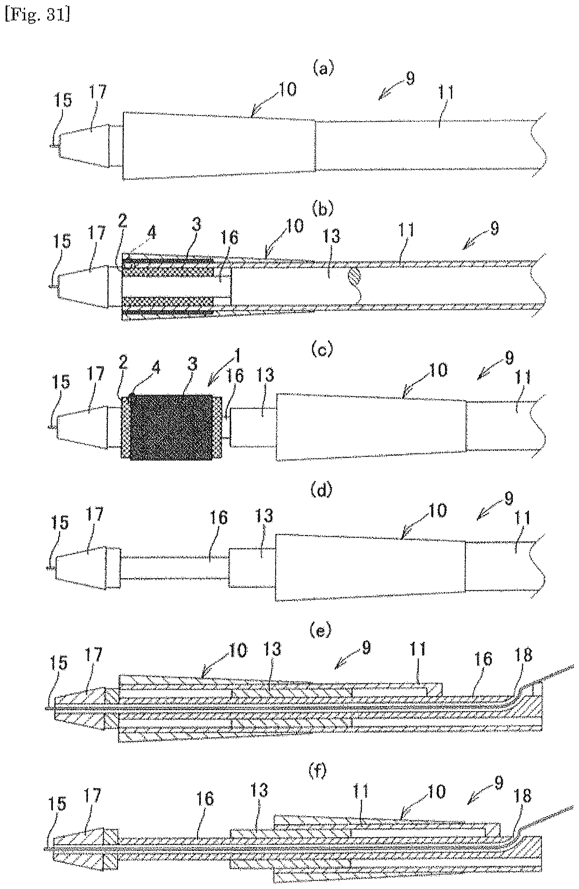

Figures 31(a) to 31(f) are views of another delivery system 9 having a protective unit 10 for delivery of self-expanding medical device. It is a delivery system wherein at least part of the main stent body and at least part of the sheet-shaped member are placed separately on the internal and external tubular surface of the delivery system.

As shown in Figure 31(a), a straight tube or a tapered tubular article is used as the protective unit 10.

Figure 31(b) shows the internal structure of the protective unit. A catheter 16 having a lumen allowing insertion of a guide wire 15 is inserted into the shaft 11 and connected to the distal end tip 17 in the distal region. The main stent body 2 of the medical device is formed externally on the catheter 16, and a sheet-shaped member 3 is inserted between the shaft 11 and the tubular article constituting the protective unit 10 and connected to the wire 4 in the distal end region. A slit may be formed on the shaft 11 close to the wire 4.

In addition, a second catheter 13 is inserted surrounding the catheter 16, and the main stent body 2 is pushed outward from the delivery system 9 when the second catheter 13 is pressed.

Figure 31(c) is a view illustrating the state in which the medical device 1 is released from the delivery system 9 shown in Figure (a). The released medical device expands by itself to a diameter of the main stent body 2 larger than the diameter of the distal end tip 17. It is possible then to indwell the medical device 1, by pulling the delivery system 9 toward the proximal side. Figure 31(d) is a view illustrating the delivery system 9 after indwelling the medical device 1.

Figures 31(e) and 31(f) are views illustrating delivery systems 9 having a catheter 16 with a lumen 18 for withdrawal of the guide wire 15 and two lumens in the cross section of the tube. In Figures 31(e) and 31(f), a releasing member 13 is fixed on the external surface of the catheter 16, replacing the second catheter. The medical device (not shown in the Figure) is placed between the distal end tip 17 and the releasing member 13. When the shaft 11 is pulled toward the proximal side, the distal region of the catheter 16 is pushed outward from the delivery system 9. In the Figure, the guide wire is inserted into the lumen of the catheter 16, but another lumen allowing insertion of the guide wire 15 may be formed outside the catheter 16.

Although not shown in Figures, the following modifications in which the configuration of each unit is modified as needed are also possible.

(1) Delivery system in which at least part of the main stent body is formed in the tube of the delivery system and at least part of the sheet-shaped member outside the tube of the delivery system
(2) Delivery system in which the protective unit is a shaft
(3) Delivery system in which the shaft penetrates into the internal side of the main stent body having the first diameter at least partially
(4) Delivery system in which at least part of the shaft is not a tubular member.

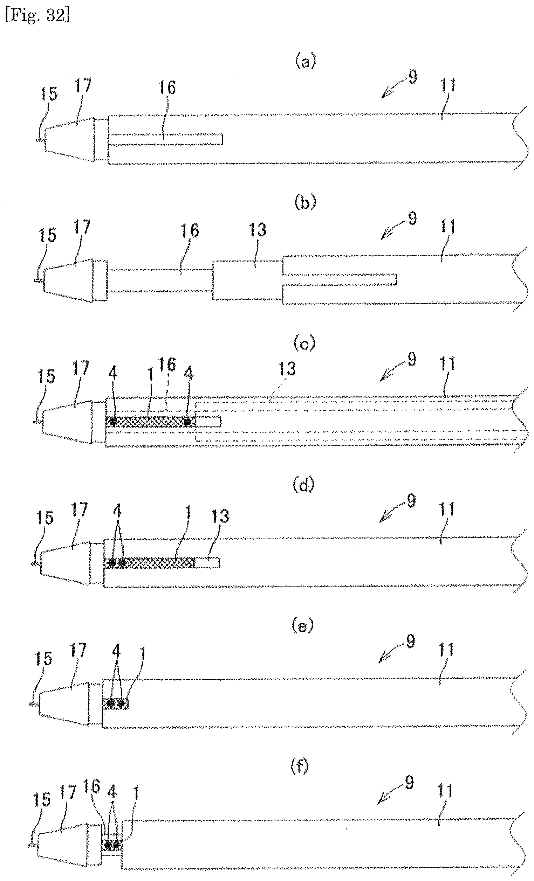

Figures 32(a) to 32(f) are views of another favorable example of the delivery system 9 for delivery of self-expanding medical device.

The delivery system (a) is a system in which no protective unit is formed and a slit is formed in the distal region of the shaft 11. Catheter 16 having a lumen allowing insertion of a guide wire 15 is inserted into the shaft 11 and connected to the distal end tip 17 in the distal region. As in the Figure, it is possible to recognize the catheter 16 visually from the slit region.

Figure (b) is a view illustrating the state in which the distal end tip 17 is pushed forward out of the delivery system 9 of Figure (a). A second catheter 13 having a larger diameter is inserted surrounding the catheter 16, and these catheters can be pressed separately from the proximal side of the delivery system. A medical device is placed in a space outside the catheter 16 and between the distal end tip 17 and the second catheter 13.

Figures (c), (d) and (e) are views of the state in which a medical device 1 is inserted into the distal region of a delivery system 9 having a slit. In Figure (c), wires 4 fixing the main stent body and the sheet-shaped member to each other (fixing sites) are placed respectively in the terminal regions of the medical device 1. In Figure (d), there are two fixing sites in the area close to the distal region. Figure (e) shows an example in which the length of the slit formed on the shaft 11 is short.

Figure (f) shows an example of the delivery system 9 in which at least part of the sheet-shaped member is placed outside the tube of the delivery system 9 and the shaft 11 has no slit.

(Example 1)

Figure 20 is a slide of the pathological sample of a dog (beagle), after indwelling a medical device, having a plain-woven metal mesh ($17\times7$ mm square, wire diameter: 0.04 mm, thickness: 0.08 mm, size of pore: 0.092 mm, pore rate: 50%, metal (SUS) content: 16.3 mg/mm$^2$) fixed on the peripheral surface of a main stent body (length: 18 mm, balloon expansion diameter: approximately 3.5 mem, made of SUS), to an aneurysm in carotid artery synthetically formed, as shown in Figure 6. Figure 20(a) shows a slide of the entire cross section of the blood vessel where the sample stent is indwelled, while Figure 20(b) is a locally expanded photograph of the slide in Figure 20(a) (x8 magnification from Figure 20(a)). It was found at one month after indwelling the medical device that conversion to endodermis was accelerated and the metal mesh 3 and the main stent body 2 were covered with endodermis entirely. Figure 20(a) was obtained at an objective magnification of $\times1.25$, and figure (b), at an objective magnification of $\times10$.

(Example 2)

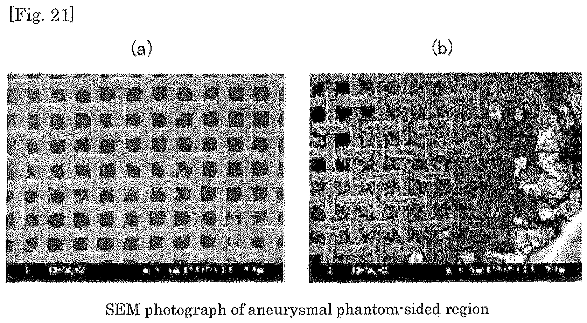

Figure 21 is photographs of a sample stent after a medical device for body cavity is placed in an aneurysmal phantom and a porcine blood is perfused. In this experiment, a medical device having a plain-woven metal mesh (17x7 mm square, wire diameter: 0.03 mm, thickness 0.05 mm, size of pore: 0.052 mm, pore rate: 43%, metal (SUS) content: 15.5 mg/mm$^2$) fixed on the peripheral surface of a main stent body (length: 18 mm, expansion diameter: approximately 3.5 mm, made of SUS) was indwelled in the aneurysmal phantom, as shown in Figure 6, and connected to porcine carotid artery for perfusion of blood for 20 minutes. The aneurysmal phantom used was a semi-spherical silicone synthetic aneurysm formed, as adhered to a. blood vessel-modeled silicone tube. Figure 21(a) is a slide of the metal mesh placed in the central region of the aneurysmal opening after perfusion test, and Figure 21(b) is a SEM photograph of the opening peripheral area of the same synthetic aneurysm in Figure 21(a). There was deposition of thrombus observed on the mesh. In particular, initiation of thrombosis was observed on the mesh in Figure 21(a), and growth of the thrombosis in Figure 21(b), Figures 21(a) and 21(b) were obtained at an objective magnification of ×150.

(Example 3)

Figure 22 is a photograph of a sample stent after a medical device for body cavity is placed in an aneurysmal phantom and a porcine blood is perfused. In this experiment, a medical device having a twill-woven metal mesh (17×7 mm square, wire diameter: 0.02 mm, thickness 0.05 mm, size of pore : 0.03 mm, pore rate: 25%, metal (SUS) content: 11.1 mg/mm$^2$) fixed on the peripheral surface of a main stent body (length: 18 mm, expansion diameter: approximately 3.5 mm, made of SUS) was indwelled in the aneurysmal phantom, as shown in Figure 6, and connected to porcine carotid artery for perfusion of blood for 20 minutes. The aneurysmal phantom used was a semi-spherical silicone synthetic aneurysm formed, as adhered to a blood vessel-modeled silicone tube.

Figure 22(a) is a photograph of the opening-sided aneurysmal phantom after perfusion experiment. Figure 22(a) showed a great amount of thrombus deposited.

Figure 22(b) is a SEM photograph of the artery phantom-sided surface of the mesh raw material after perfusion experiment (magnification: ×40), and Figure 22(c) is an expanded photograph thereof (×10 magnification from Figure 22(b)).

Figures 22(b) and 22(c) showed deposition of a great amount of thrombus sufficient to cover the aneurysmal opening-sided mesh opening region and the filament material, 20 minutes after perfusion.

Figure 22(d) is a SEM photograph of the synthetic blood vessel-sided surface of the mesh raw material after perfusion test, and Figure 22(e) is an expanded photograph thereof(×9 magnification from Figure 22(d)). Figures 22(b) to 22(e) showed deposition of thrombus in the aneurysmal phantom side in an amount greater than synthetic blood vessel side, indicating that accelerated thrombosis of aneurysm and conversion to endodermis even in a short period of 20 minutes. Figure 21(a) is an optical photograph, and Figure 21(b) is obtained at an objective magnification of ×40; Figure 21(c), of ×400; Figure 21(d), of ×40; and Figure 21(e) of ×350.

(Example 4)

Figure 23 is a SEM photograph of the mesh region of a medical device when the medical device for body cavity is placed in an aneurysmal phantom and porcine blood was perfused. In this experiment, a medical device having a plain-woven metal mesh (16x8 mm square, wire diameter: 0.013 mm, thickness 0.03 mm, size of pore: 0.03 mm, pore rate: 49%, metal (SUS) content: 5.4 mg/mm$^2$) fixed on the peripheral surface of a main stent body (length: 18 mm, expansion diameter: approximately 3.5 mm, made of SUS) was indwelled in the aneurysmal phantom, as shown in Figure 6, and connected to porcine carotid artery for perfusion of blood for 20 minutes. The aneurysmal phantom used was a semi-spherical silicone synthetic aneurysm formed, as adhered to a blood vessel-modeled silicone tube.

Figure 23(a) is a SEM photograph of the aneurysmal phantom opening-sided surface of the mesh raw material after perfusion test, and Figure 23(b) is an expanded photograph thereof (x3.3 magnification from Figure 23(a)).

Figures 23(a) and 23(b) showed that deposition of a great amount of thrombus sufficient to cover the aneurysmal opening-sided mesh opening region and the filament material, 20 minutes after perfusion.

Figure 23(c) is a SEM photograph of the synthetic blood vessel-sided surface of the mesh raw material after perfusion test, and Figure 23(d) is an expanded photograph thereof ×0.27 magnification from Figure 23(c)).

Figures 23(a) to 23(d) showed deposition of thrombus in the aneurysmal phantom side in an amount greater than synthetic blood vessel side, indicating that accelerated thrombosis of aneurysm and conversion to endodermis even in a short period of 20 minutes, similarly to Figure 22. Figure 23(a) is obtained at an objective magnification of ×150; Figure 23(b), of ×500; Figure 23(c), of ×40; and Figure 23(d), of ×300.

(Example 5)

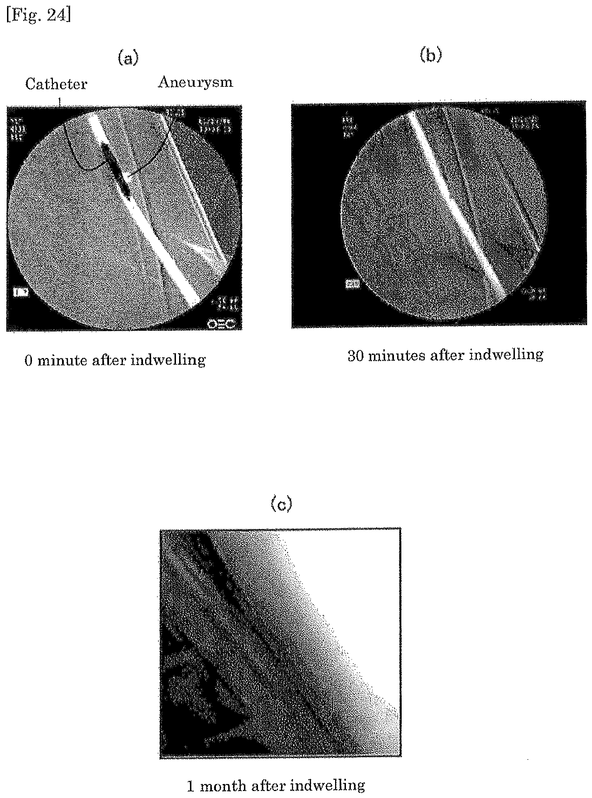

Figure 24 shows radiographs of the pathological samples of a dog, before and after indwelling and one month after indwelling a medical device having a plain-woven metal mesh (16×16 mm square, wire diameter,: 0.013 mm, thickness: 0.03 mm, size of pore: 0.03 mm, pore rate: 49%, metal (SUS) content: 5.4 mg/mm$^2$) fixed on the peripheral surface of a main stent body (length: 18 mm, balloon expansion diameter: approximately 3.5 mm, made of Co-Cr), as shown in Figure 7(b), to the aneurysm in carotid artery synthetically formed. The plain-woven metal mesh used was the same as that used in Figure 23. Figure 24(a) is the aneurysmal radiograph before indwelling; Figure 24(b), 30 minutes after indwelling; and Figure 24(c), 1 month after indwelling. There was almost no flow of the contrast medium into the aneurysm at 30 minutes after indwelling, indicating accelerated thrombosis. The aneurysmal opening was apparently blocked in one month.

As shown in Figure 24(c), the blood flowed uninterruptedly in the blood vessel containing the indwelled medical device, and there was no observed trouble such as constriction.

(Example 6)

Figures 33(a) to 33(c) are radiographs (photographs and autopsy result) of the pathological samples of a dog before and after indwelling and two month after indwelling a medical device having a twill-woven metal mesh (17×7 mm square, wire diameter: 0.02 mm, thickness: 0.05 mm, size of pore: 0.03 mm, pore rate: 25%, metal (SUS) content: 11.1 mg/mm$^2$) fixed on the peripheral surface of a main stent body (length: 18 mm, balloon expansion diameter: approximately 3.5 mm, made of Co-Cr), as shown in Figure 7(b), to the aneurysm in carotid artery synthetically formed. The twill-woven metal mesh used was the same as that used in Figure 22. Figure 33(a) is an aneurysmal radiograph before indwelling; and Figure 33(b) is an aneurysmal radiograph 5 minutes after indwelling. There was almost no flow of the contrast medium into the aneurysm observed from immediately after indwelling, and observation the aneurysmal region and the blood vessel-containing organ after cleavage showed, as shown in Figure 33 (c), that the blood vessel region had a cavity, while the aneurysmal region of the lower blood vessel in Figure was converted to endodermis. Figure 33(c) was a radiograph obtained at a magnification of ×1.25.

(Example 7)

Figures 34(a) and 34(b) are radiographs of the pathological samples of a dog, before and after indwelling a medical device having a plain-woven metal mesh (16×16 mm square, wire diameter: 0.03 mm, thickness: 0.03 mm, size of pore: 0.03 mm, pore rate: 49%, metal (SUS) content: 5.4 mg/mm$^2$) fixed on the peripheral surface of a main stent body (length: 18 mm, stent diameter: approximately 4.0 mm, made of Ni-Ti), as shown in Figure 29, to the aneurysm in carotid artery synthetically formed. The plain-woven metal mesh was the same as that used in Figure 23. The delivery system had the same configuration as those in Figures 27 to 30, and the plain-woven metal mesh was previously molded into the shape shown in Figure 26. Figure 34(a) is a radiograph before indwelling, and Figure 34(b) is a radiograph 5 minutes after indwelling. There was almost no flow of the contrast medium into the aneurysm observed, 5 minutes after indwelling.

(Example 8)

In the test, a medical device having a plain-woven metal mesh (17×7 mm. square, wire diameter: 0.04 mm, thickness: 0.08 mm, size of pore: 0.092 mm, pore rate: 50%, metal (SUS316L) content: 16.3 mg/mm$^2$) fixed on the peripheral surface of a main stent body (length: 18 mm, expansion diameter: approximately 3.5 mm, made of SUS316L), as shown in Figure 6, was indwelling in an aneurysmal phantom, and the aneurysmal phantom was connected to the porcine carotid artery, allowing perfusion of the blood for 30 minutes. There was no obstruction observed after perfusion for 30 minutes. The aneurysmal phantom used was a semi-spherical silicone synthetic aneurysm formed, as adhered to a blood vessel-modeled silicone tube. Figures 35(a) and 35(b) are SEM photographs of the aneurysmal phantom side of the sample after test. Figure 35(a) was obtained at an objective magnification of ×10, and Figure (b), at an objective magnification of ×150. Figure 35(b) shows that there is deposition of various cells observed on the mesh surface after perfusion for 30 minutes, but apparently without obstruction or without any tendency to obstruction.

The materials for the medical devices used in Examples 1 to 8 are summarized in Table 2.

[Table 2]

| SUS materials | | | | | | | | |
|---|---|---|---|---|---|---|---|---|
| | Example 1 | Example2 | Example3 | Examples4 | Example5 | Example6 | Example7 | Example8 |
| Stent | SUS316L | SUS316L | SUPS3161 | SUS316L | Co-Cr | Co-Cr | Ni-Ti | SUS316L |
| Mesh | SUPS316L | SUS304 | SUS304 | SUS316 | SUS316 | SUS304 | SUS316 | SUS316L |

An action similar to that to the aneurysm described in the Examples above is expected to other diseases associated with opening of other celomic cavity such as varicosis and microaneurysm.

(Preparative Examples for medical devices)

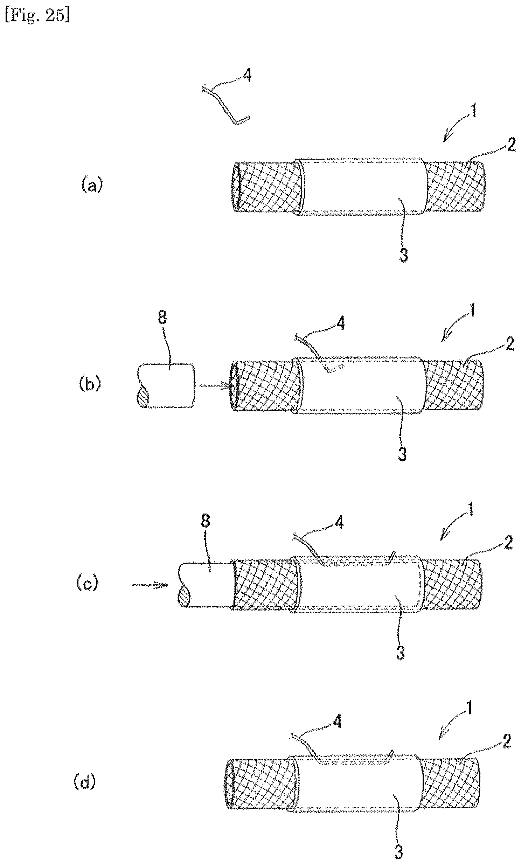

The medical devices used in Examples 1 to 4 were prepared according to the procedure shown in Figure 25:

Step (a): a wire 4 with an angle in the distal region and a main stent body 2 having a sheet-shaped member 3 located at a particular site are made available.

Step (b): then, the wire 4 with an angle in the distal region is fed through the sheet-shaped member 3 and the main stent body 2, from the external surface side to the internal surface side of the main stent body 2 having a sheet-shaped member 3 located at a particular site.

Step (c): then, the distal region of the wire 4 is pushed to the internal wall of the main stent body 2 by insertion of a rod 8 having a diameter similar to the stent internal diameter along the internal peripheral side of the main stent body 2, making the distal region of the angled wire 4 advance once again through the main stent body 2 and the sheet-shaped member 3. The wire 4 need not penetrate the main stent body 2 and the sheet-shaped member 3 simultaneously, and may penetrate separately if it is easier in production. During penetration thereof through the main stent body 2 and the sheet-shaped member 3, the sheet-shaped member 3 may or may not be formed in a tubular shape along the external surface of the main stent body 2. The direction of insertion of the rod 8 should be identical with the penetration direction of the wire 4. The diameter of the rod 8 need not be the same as the internal diameter of the main stent body 2, and is not particularly limited, if the rod can push the distal region of the wire 4 outward.

Step (d): the rod 8 is withdrawn from the internal peripheral side of the main stent body 2, and the distal region of the penetrated wire 4 is withdrawn for example with tweezers, for completion of sewing. The terminal of the sewn wire may be welded or knotted.

If the sewing is repeated multiple times, the steps (a) to (d) are repeated. The form of the wire 4 penetrating the sheet-shaped member 3 and the main stent body 2 is shown in Figure 14(c) where the sheet-shaped member 3 is as shown in Figure 7(a).

The medical device used in Example 5 was prepared, as shown in Figure 7(a), by feeding a wire 4 into a sheet-shaped member 3 with tweezers and bonding the wire 4 and the main stent body 2 by welding (laser welding).

Industrial Applicability

The medical device according to the present invention is applicable to aneurysm, varicosis and microaneurysm, particularly to intacranial aneurysm, cerebral artery after carotid siphon, and aneurysm of communicating artery, and can be used favorably in treatment of these diseases.

In the following, some embodiments of the present invention are summarized:

1. A medical device for body cavity, comprising
a main stent body and
a sheet-shaped member having pores and covering at least part of the main stent body,

2. The medical device for body cavity according to item 1, wherein,
when the main stent body is expanded from a compressed first diameter to a second diameter, the sheet-shaped member spreads in such a manner that the sheet-shaped member does not restrict the movement of the main stent body in the circumferential direction and covers at least part of the main stent body, and the shape of the pores of the sheet-shaped member is preserved even after expansion of the main stent body.

3. The medical device for body cavity according to item 2, wherein the sheet-shaped member is a sheet-shaped member having a fine structure and the fine structure of the sheet-shaped member is preserved without change even after expansion of the main stent body.

4. The medical device for body cavity according to any one of items 1 to 3, comprising a main stent body formed in a tubular structure expandable from a compressed first diameter to a second diameter and
a sheet-shaped member having pores and covering at least part of the main stent body, wherein
when the main stent body is expanded, the sheet-shaped member spreads in such a manner that the sheet-shaped member does not restrict the movement of the main stent body in the circumferential direction and covers at least part of the main stent body and the shape of the pores of the sheet-shaped member is preserved even after expansion of the main stent body.

5. The medical device for body cavity according to any one of items 1 to 3, comprising a main stent body formed in a tubular structure and expandable in the circumferential direction from a compressed first diameter to a second diameter and a fine-structured sheet-shaped member having multiple pores, wherein the sheet-shaped member spreads, as it covers at least part of the main stent body, together with the expansion of the main stent body in the circumferential direction when the main stent body is expanded and the fine structure of the sheet-shaped member is preserved without change even after expansion of the main stent body.

6. The medical device for body cavity according to any one of items 1 to 5, wherein the sheet-shaped member is fixed to a region of the main stent body.

7. The medical device for body cavity according to item 6, wherein the sheet-shaped member is fixed to the region of the main stent body in such a manner that the size of the pores is preserved substantially after expansion of the main stent body.

8. The medical device for body cavity according to item 6, wherein the fixing member is a wire.

9. The medical device for body cavity according to item 6, wherein the sheet-shaped member is fixed at one point.

10. The medical device for body cavity according to item 9, wherein the sheet-shaped member is fixed only to the distal region of the main stent body.

11. The medical device for body cavity according to item 10, wherein the sheet-shaped member is fixed to the most distal region of the main stent body.

12. The medical device for body cavity according to item 6, wherein the sheet-shaped member is fixed to the peripheral surface of the main stent body at two or more points on a line in parallel with the axial direction of the main stent body region.

13. The medical device for body cavity according to item 11, wherein the sheet-shaped member is fixed to the main stent body region, with a fixing member having an element in parallel with the axial direction of the main stent body region.

14. The medical device for body cavity according to item 12, wherein the sheet-shaped member is sewn with a wire.

15. The medical device for body cavity according to any one of items 6 to 14, wherein the sheet-shaped member and the main stent body are fixed to each other with a metal or polymeric material.

16. The medical device for body cavity according to item 15, wherein the polymeric material is a polyvinylalcohol (PVA), a cyanoacrylate, a biodegradable material or a glycolic polylactate acid copolymer (PLGA).

17. The medical device for body cavity according to any one of items 1 to 16, wherein the main stent body has a length larger than that of the sheet-shaped member.

18. The medical device for body cavity according to any one of items 2 to 5, wherein the internal diameter of the main stent body is 0,01 to 0,04 inch (0.25 to 1.02 mm) when it has a compressed first diameter.

19. The medical device for body cavity according to any one of items 1 to 5, wherein the main stent body is expanded in the shape of straight tube.

20. The medical device for body cavity according to any one of items 1 to 5, wherein the main stent body is expanded into a tapered shape.

21. The medical device for body cavity according to item 6, wherein at least part of the main stent body has a structure for fixation of the sheet-shaped member.

22. The medical device for body cavity according to any one of items 1 to 21, wherein the sheet-shaped member contains an X-ray-impermeable filament material at least partially.

23. The medical device for body cavity according to any one of items 6 to 22, wherein the sheet-shaped member contains an X-ray-impermeable material at the fixing site.

24. The medical device for body cavity according to any one of items 2 to 5, wherein the medical device contains the sheet-shaped member in which the first and second terminals of the sheet-shaped member overlap each other, when the main stent body region has the first diameter.

25. The medical device for body cavity according to item 24, wherein the sheet-shaped member overlaps the circumference of the stent in an angular range of 340° or less.

26. The medical device for body cavity according to item 24 or 25, wherein the sheet-shaped member covers the entire circumference of the main stent body region, when the main stent body region has the second diameter.

27. The medical device for body cavity according to item 24 or 25, wherein the sheet-shaped member covers the circumference of the stent circumference in an angular range of 60° or more and 300° or less, when the main stent body region has the second diameter.

28. The medical device for body cavity according to any one of items 1 to 27, wherein the sheet-shaped member is a mesh.

29. The medical device for body cavity according to item 28, wherein the mesh is formed by weaving a filament material.

30. The medical device for body cavity according to item 29, wherein the wire diameter of the filament material of the sheet-shaped member is smaller than the thickness of the main stent body region.

31. The medical device for body cavity according to item 29 or 30, wherein the wire diameter of the filament material is 0.2 mm or less.

32. The medical device for body cavity according to any one of items 28 to 31, wherein the size of the pores of the sheet-shaped member is 0.2 mm or less.

33. The medical device for body cavity according to any one of items 1 to 32, wherein the pore rate of the sheet-shaped member is 50% or less.

34. The medical device for body cavity according to any one of items 1 to 33, wherein the average thickness of the sheet-shaped member is 0.2 mm or less.

35. The medical device for body cavity according to any one of items 1 to 34, wherein the material for the sheet-shaped member is a metal.

36. The medical device for body cavity according to item 35, wherein the metal content of the sheet-shaped member is 16.3 mg/mm$^2$ or less.

37. The medical device for body cavity according to item 35, wherein the material for the sheet-shaped member is SUS or SUS316,

38. The medical device for body cavity according to any one of items 29 to 37, wherein the sheet-shaped member is made of plain weave or twilled weave.

39. The medical device for body cavity according to any one of items 2 to 5, wherein the curvature memorized by the sheet-shaped member is different from the curvature of the main stent body in the second diameter.

40. The medical device for body cavity according to any one of items 2 to 5, wherein at least part of the sheet-shaped member has a curvature in a direction different from that of the main stent body in the second diameter.

41. The medical device for body cavity according to any one of items 2 to 5, wherein the sheet-shaped member has folding lines at least partially.

42. The medical device for body cavity according to any one of items 2 to 5, wherein the sheet-shaped member memorizes waveform.

43. The medical device for body cavity according to any one of items 1 to 42, wherein the materials for the main stent body and the sheet-shaped member are the same.

44. The medical device for body cavity according to item 43, wherein the same material for the main stent body and the sheet-shaped member is a metal.

45. A method of producing the medical device according to any one of items 8 to 44, comprising a sewing step of making the wire have an angle in the terminal region, placing the sheet-shaped member at a particular position, sending the wire, as it penetrates through the sheet-shaped member and the main stent body from the external surface side to the internal peripheral side of main stent body, and sending the wire terminal region from the stent internal peripheral side to the external surface side by feeding a rod having a diameter similar to the diameter of the stent internal diameter into the internal peripheral side of the main stent body.

46. A delivery system, comprising the medical device for body cavity according to any one of items 1 to 45 and a first catheter for feeding the medical device for body cavity into the body, wherein the medical device for body cavity is located in the distal region of the first catheter so that the medical device can be indwelled.

47. The delivery system according to item 46, wherein at least part of the sheet-shaped member is placed separately from the main stent body.

48. The delivery system according to item 47, wherein the first catheter has a first tubular member, the main stent body having a compressed first diameter can be placed internally at the distal end of the first tubular member, and the sheet-shaped member can be placed externally at the distal end of the first tubular member.

49. The delivery system according to item 47, wherein the first catheter has a first tubular member forming the catheter main body and a second tubular member placed externally on the first tubular member, and the main stent body having a compressed first diameter can be placed internally at the distal end of the first tubular member and the sheet-shaped member can be placed between the first and second tubular members.

50. The delivery system according to item 48 or 49, wherein the first tubular member has at least one penetrating lumen at the distal end.

51. The delivery system according to item 48 or 49, wherein the first tubular member has a hole communicating with the lumen at least at one position on the outermost surface.

52. The delivery system according to any one of items 48 to 51, wherein the first tubular member has a slit at the distal end.

53. The delivery system according to any one of items 48 to 51, wherein the second tubular member is the protective unit of the sheet-shaped member.

54. The delivery system according to item 53, wherein the protective unit is a thin film.

55. The delivery system according to item 54, wherein the thin film is made of a polymer compound material.

56. The delivery system according to any one of items 53 to 55, wherein the internal diameter of the protective unit is 1.5 to 2.5 mm.

57. The delivery system according to any one of items 53 to 56, wherein the protective unit is fixed to the first tubular member.

58. The delivery system according to any one of items 53 to 57, wherein the external shape of the protective unit is a tapered shape.

59. The delivery system according to any one of items 46 to 58, wherein the main stent body region having a compressed first diameter is placed in the most internal lumen of the first tubular member and the sheet-shaped region is placed between the first and second tubular members.

60. The delivery system according to any one of items 46 to 59, further comprising a second catheter, penetrating inserted in the first tubular member so as to push and for use in pushing the medical device for body cavity from the rear edge side.

61. The delivery system according to item 60, wherein the second catheter is tubular in shape and its internal diameter is 0.01 to 0.04 inch (0.25 to 1,02 mm).

62. The delivery system according to any one of items 46 to 61, wherein the first or second catheter retains the internal diameter allowing absorption after indwelling the medical device.


**Claims**

1.  A medical device (1) for body cavity, comprising
    a main stent body (2) and a sheet-shaped member (3) having pores and covering at least part of the main stent body (2),
    **characterized in that** the sheet-shaped member (3) is fixed only to the most distal region of the main stent body (2) at one point.

2.  The medical device (1) for body cavity according to claim 1, wherein,
    when the main stent body (2) is expanded from a compressed first diameter to a second diameter, the sheet-shaped member (3) spreads in such a manner that the sheet-shaped member (3) does not restrict the movement of the main stent body (2) in the circumferential direction and covers at least part of the main stent body (2), and the shape of the pores of the sheet-shaped member (3) is preserved even after expansion of the main stent body (2).

3.  The medical device (1) for body cavity according to claim 2, wherein the sheet-shaped member (3) is a sheet-shaped member (3) having a fine structure which is a structure having pores in a particular shape and the fine structure of the sheet-shaped member (3) is preserved without change even after expansion of the main stent body (2).

4.  The medical device (1) for body cavity according to any one of claims 1 to 3, comprising a main stent body (2) formed in a tubular structure and expandable in the circumferential direction from a compressed first diameter to a second diameter and a fine-structured sheet-shaped member (3) having multiple pores, wherein the sheet-shaped member (3) spreads, as it covers at least part of the main stent body (2), together with the expansion of the main stent body (2) in the circumferential direction when the main stent body (2) is expanded and the fine structure of the sheet-shaped member (3) is preserved without change even after expansion of the main stent body (2).

5.  The medical device (1) for body cavity according to claim 1, wherein the sheet-shaped member (3) is fixed to the region of the main stent body (2) in such a manner that the size of the pores is preserved substantially after expansion of the main stent body (2).

6.  The medical device (1) for body cavity according to claim 1, wherein the fixing member is a wire (4).

7.  The medical device (1) for body cavity according to claim 1, wherein the sheet-shaped member (3) is fixed to the main stent body (2) region, with a fixing member having an element in parallel with the axial direction of the main stent body (2) region.

8.  The medical device (1) for body cavity according to claim 1, wherein at least part of the main stent body (2) has a structure for fixation of the sheet-shaped member (3).

9.  The medical device (1) for body cavity according to any one of claims 1 to 8, wherein the sheet-shaped member (3) contains an X-ray-impermeable filament material at least partially.

**10.** The medical device (1) for body cavity according to any one of claims 1 and 5 to 9, wherein the sheet-shaped member (3) contains an X-ray-impermeable material at the fixing site.

**11.** The medical device (1) for body cavity according to any one of claims 2 to 4, wherein the medical device (1) contains the sheet-shaped member (3) in which the first and second terminals of the sheet-shaped member (3) overlap each other, when the main stent body (2) region has the first diameter.

**12.** The medical device (1) for body cavity according to any one of claims 1 to 11, wherein the sheet-shaped member (3) is a mesh.

**13.** The medical device (1) for body cavity according to claim 6, wherein the sheet-shaped member (3) is a mesh and the mesh is formed by weaving a filament material, and wherein the wire (4) diameter of the filament material of the sheet-shaped member (3) being the mesh is smaller than the thickness of the main stent body (2) region.

**14.** The medical device (1) for body cavity according to any one of claims 12 or 13, wherein the size of the pores of the sheet-shaped member (3) is 0.2 mm or less.

**15.** The medical device (1) for body cavity according to any one of claims 1 to 14, wherein the material for the sheet-shaped member (3) is a metal.

**16.** The medical device (1) for body cavity according to any one of claims 2 to 4, wherein the curvature of the sheet-shaped member (3) is different from the curvature of the main stent body (2) in the second diameter.

**17.** A method of producing the medical device (1) according to any one of claims 6 to 16, comprising a sewing step of making the wire (4) have an angle in the terminal region, placing the sheet-shaped member (3) at a particular position, sending the wire (4), as it penetrates through the sheet-shaped member (3) and the main stent body (2) from the external surface side to the internal peripheral side of main stent body (2), and sending the wire (4) terminal region from the stent internal peripheral side to the external surface side by feeding a rod (8) having a diameter similar to the diameter of the stent internal diameter into the internal peripheral side of the main stent body (2).

**18.** A delivery system, comprising the medical device (1) for body cavity according to any one of claims 1 to 16 and a first catheter for feeding the medical device (1) for body cavity into the body, wherein the medical device (1) for body cavity is located in the distal region of the first catheter so that the medical device (1) can be indwelled.

**19.** The delivery system according to claim 18, wherein at least part of the sheet-shaped member (3) is placed separately from the main stent body (2), **characterized in that** the first catheter has a first tubular member (11), the main stent body (2) having a compressed first diameter can be placed internally at the distal end of the first tubular member (11), and the sheet-shaped member (3) can be placed externally at the distal end of the first tubular member (11).

**20.** The delivery system according to claim 19, wherein the first catheter has a first tubular member (11) forming the catheter main body and a second tubular member (10) placed externally on the first tubular member (11), and the main stent body (2) having a compressed first diameter can be placed internally at the distal end of the first tubular member (11) and the sheet-shaped member (3) can be placed between the first and second tubular members (10, 11).

**21.** The delivery system according to any one of claims 19 to 20, wherein the first tubular member (11) has a slit at the distal end.

**22.** The delivery system according to any one of claims 18 to 20, further comprising a second catheter (13), penetrating inserted in the first tubular member (11) so as to push and for use in pushing the medical device (1) for body cavity from the rear edge side.

**23.** The delivery system according to any one of claims 18 to 22, wherein the first or second catheter (13) retains the internal diameter allowing absorption after indwelling the medical device.

**Patentansprüche**

**1.** Medizinische Vorrichtung (1) für eine Körperhöhle, umfassend

einen Hauptstentkörper (2) und ein plattenförmiges Element (3), welches Poren aufweist und mindestens einen Teil des Hauptstentkörpers (2) bedeckt, **dadurch gekennzeichnet, dass** das plattenförmige Element (3) nur am distalsten Bereich des Hauptstentkörpers (2) an einer Stelle befestigt ist.

2.  Die medizinische Vorrichtung (1) für eine Körperhöhle nach Anspruch 1, wobei, wenn der Hauptstentkörper (2) von einem komprimierten ersten Durchmesser zu einem zweiten Durchmesser expandiert wird, das plattenförmige Element (3) sich derartig aufspreizt, dass das plattenförmige Element (3) nicht die Bewegung des Hauptstentkörpers (2) in Umfangsrichtung einschränkt und mindestens einen Teil des Hauptstentkörpers (2) bedeckt, und die Form der Poren des plattenförmigen Elements (3) auch nach der Expansion des Hauptstentkörpers (2) erhalten bleibt.

3.  Die medizinische Vorrichtung (1) für eine Körperhöhle nach Anspruch 2, wobei das plattenförmige Element (3) ein plattenförmiges Element (3) ist, welches eine feine Struktur aufweist, wobei die Struktur Poren in einer bestimmten Form ist, und die feine Struktur des plattenförmigen Elements (3) auch nach der Expansion des Hauptstentkörpers (2) ohne Veränderung erhalten bleibt.

4.  Die medizinische Vorrichtung (1) für eine Körperhöhle nach einem der Ansprüche 1 bis 3, umfassend einen Hauptstentkörper (2), der in einer röhrenförmigen Struktur ausgebildet ist und der in Umfangsrichtung von einem komprimierten ersten Durchmesser zu einem zweiten Durchmesser expandierbar ist, und ein plattenförmiges Element (3) mit feiner Struktur, welches eine Vielzahl an Poren aufweist, wobei das plattenförmige Element (3), welches mindestens einen Teil des Hauptstentkörpers (2) bedeckt, sich zusammen mit der Expansion des Hauptstentkörpers (2) in Umfangsrichtung aufspreizt, wenn der Hauptstentkörper (2) expandiert wird und die feine Struktur des plattenförmigen Elements (3) auch nach der Expansion des Hauptstentkörpers (2) ohne Veränderung erhalten bleibt.

5.  Die medizinische Vorrichtung (1) für eine Körperhöhle nach Anspruch 1, wobei das plattenförmige Element (3) derartig an dem Bereich des Hauptstentkörpers (2) befestigt ist, dass die Porengröße nach der Expansion des Hauptstentkörpers (2) im Wesentlichen ohne Veränderung erhalten bleibt.

6.  Die medizinische Vorrichtung (1) für eine Körperhöhle nach Anspruch 1, wobei das Fixierungselement ein Draht (4) ist.

7.  Die medizinische Vorrichtung (1) für eine Körperhöhle nach Anspruch 1, wobei das plattenförmige Element (3) an dem Bereich des Hauptstentkörpers (2) mit einem Fixierungselement, welches ein Element parallel zur Axialrichtung des Bereichs des Hauptstentkörpers (2) aufweist, befestigt ist.

8.  Die medizinische Vorrichtung (1) für eine Körperhöhle nach Anspruch 1, wobei mindestens ein Teil des Hauptstentkörpers (2) eine Struktur zur Fixierung des plattenförmigen Elements (3) aufweist.

9.  Die medizinische Vorrichtung (1) für eine Körperhöhle nach einem der Ansprüche 1 bis 8, wobei das plattenförmige Element (3) zumindest teilweise ein für Röntgenstrahlen undurchlässiges Fasermaterial enthält.

10. Die medizinische Vorrichtung (1) für eine Körperhöhle nach einem der Ansprüche 1 und 5 bis 9, wobei das plattenförmige Element (3) ein für Röntgenstrahlen undurchlässiges Material an der Befestigungsstelle enthält.

11. Die medizinische Vorrichtung (1) für eine Körperhöhle nach einem der Ansprüche 2 bis 4, wobei die medizinische Vorrichtung (1) das plattenförmige Element (3) enthält, in dem der erste und der zweite Anschluss des plattenförmigen Elements (3) sich überlappen, wenn der Bereich des Hauptstentkörper (2) den ersten Durchmesser aufweist.

12. Die medizinische Vorrichtung (1) für eine Körperhöhle nach einem der Ansprüche 1 bis 11, wobei das plattenförmige Element (3) ein Maschennetz ist.

13. Die medizinische Vorrichtung (1) für eine Körperhöhle nach Anspruch 6, wobei das plattenförmige Element (3) ein Maschennetz ist und das Maschennetz durch Weben eines Fasermaterial gebildet wird, und wobei der Drahtdurchmesser des Fasermaterials des plattenförmigen Elements (3), das das Maschennetz ist, kleiner ist als die Dicke des Bereichs des Hauptstentkörpers (2).

14. Die medizinische Vorrichtung (1) für eine Körperhöhle nach Anspruch 12 oder 13, wobei die Porengröße des plattenförmigen Elements (3) 0,2 mm oder weniger ist.

**15.** Die medizinische Vorrichtung (1) für eine Körperhöhle nach einem der Ansprüche 1 bis 14, wobei das Material für das plattenförmige Element (3) ein Metall ist.

**16.** Die medizinische Vorrichtung (1) für eine Körperhöhle nach einem der Ansprüche 2 bis 4, wobei die Krümmung des plattenförmigen Elements (3) anders ist als die Krümmung des Hauptstentkörpers (2) beim zweiten Durchmesser.

**17.** Verfahren zur Herstellung der medizinischen Vorrichtung nach einem der Ansprüche 6 bis 16, umfassend einen Nähschritt aus Erstellen eines Drahts (4) mit einem Winkel im Endbereich, Platzieren des plattenförmigen Elements (3) an einer bestimmten Position, Führen des Drahts (4), welcher das plattenförmige Element (3) und den Hauptstentkörper (2) von der Außenflächenseite zur Innenumfangsseite des Hauptstentkörper (2) durchdringt, und Führen des Endbereichs des Drahts (4) von der Innenumfangsseite des Stens über die Außenflächenseite durch Zuführen eines Stabs (8) mit einem Durchmesser, der dem Durchmesser des internen Stentdurchmessers ähnlich ist, zur Innenumfangsseite des Hauptstentkörper (2).

**18.** Zuführsystem, umfassend die medizinische Vorrichtung (1) für eine Körperhöhle nach einem der Ansprüche 1 bis 16 und ein erster Katheter zum Einführen der medizinischen Vorrichtung (1) für eine Körperhöhle in den Körper, wobei die medizinische Vorrichtung (1) für eine Körperhöhle im distalen Bereich des ersten Katheters angeordnet ist, damit die medizinische Vorrichtung (1) eingepflanzt werden kann.

**19.** Das Zuführsystem nach Anspruch 18, wobei mindestens ein Teil des plattenförmigen Elements (3) getrennt vom Hauptstentkörper (2) angeordnet ist, **dadurch gekennzeichnet, dass** der erste Katheter ein erstes röhrenförmiges Element (11) aufweist, und der Hauptstentkörper (2) mit einem komprimierten ersten Durchmesser innenseitig am distalen Ende des ersten röhrenförmigen Elements (11) angeordnet werden kann, und das plattenförmige Element (3) außenseitig am distalen Ende des ersten röhrenförmigen Elements (11) angeordnet werden kann.

**20.** Das Zuführsystem nach Anspruch 19, wobei das erste Katheter ein erstes röhrenförmiges Element (11), welches den Hauptkörper des Katheters bildet, und ein zweites röhrenförmiges Element (10), welches außenseitig auf dem ersten röhrenförmigen Element (11) angeordnet ist, aufweist, und der Hauptstentkörper (2) mit einem komprimierten ersten Durchmesser innenseitig am distalen Ende des ersten röhrenförmigen Elements (11) angeordnet werden kann, und das plattenförmige Element (3) zwischen dem ersten und zweiten röhrenförmigen Element (10, 11) angeordnet werden kann.

**21.** Das Zuführsystem nach Anspruch 19 oder 20, wobei das erste röhrenförmige Element (11) einen Schlitz am distalen Ende aufweist.

**22.** Das Zuführsystem nach einem der Ansprüche 18 bis 20, welches zusätzlich einen zweiten Katheter (13) umfasst, welcher zum Verschieben durchdringend in das erste röhrenförmige Element (11) eingebracht ist, und zur Verwendung beim Verschieben der medizinischen Vorrichtung (1) für eine Körperhöhle von der Hinterkantenseite.

**23.** Das Zuführsystem nach einem der Ansprüche 18 bis 22, wobei der erste oder der zweite Katheter (13) den Innendurchmesser beibehält, um eine Absorption nach dem Einpflanzen der medizinischen Vorrichtung zu ermöglichen.

**Revendications**

**1.** Dispositif médical (1) pour une cavité corporelle, comprenant
un corps d'endoprothèse principal (2) et un organe en forme de feuille (3) ayant des pores et couvrant au moins une partie du corps d'endoprothèse principal (2),
**caractérisé en ce que** l'organe en forme de feuille (3) n'est fixé qu'en un point à la région la plus distale du corps d'endoprothèse principal (2).

**2.** Dispositif médical (1) pour une cavité corporelle selon la revendication 1, dans lequel,
lorsque le corps d'endoprothèse principal (2) est déployé d'un premier diamètre comprimé à un deuxième diamètre, l'organe en forme de feuille (3) s'étend de sorte que l'organe en forme de feuille (3) ne limite pas le mouvement du corps d'endoprothèse principal (2) dans la direction circonférentielle et couvre au moins une partie du corps d'endoprothèse principal (2), et la forme des pores de l'organe en forme de feuille (3) est conservée même après déploiement du corps d'endoprothèse principal (2).

**3.** Dispositif médical (1) pour une cavité corporelle selon la revendication 2, dans lequel l'organe en forme de feuille (3) est un organe en forme de feuille (3) ayant une structure fine qui est une structure présentant des pores dans une forme particulière et la structure fine de l'organe en forme de feuille (3) est conservée sans changement même après déploiement du corps d'endoprothèse principal (2).

**4.** Dispositif médical (1) pour une cavité corporelle selon l'une quelconque des revendications 1 à 3, comprenant un corps d'endoprothèse principal (2) se présentant sous la forme d'une structure tubulaire et pouvant se déployer dans la direction circonférentielle d'un premier diamètre comprimé à un deuxième diamètre et un organe en forme de feuille (3) à structure fine présentant de multiples pores, où l'organe en forme de feuille (3) s'étend, comme il couvre au moins une partie du corps d'endoprothèse principal (2), conjointement avec le déploiement du corps d'endoprothèse principal (2) dans la direction circonférentielle lorsque le corps d'endoprothèse principal (2) est déployé et la structure fine de l'organe en forme de feuille (3) est conservée sans changement même après déploiement du corps d'endoprothèse principal (2).

**5.** Dispositif médical (1) pour une cavité corporelle selon la revendication 1, dans lequel l'organe en forme de feuille (3) est fixé à la région du corps d'endoprothèse principal (2) de sorte que la taille des pores soit essentiellement conservée après déploiement du corps d'endoprothèse principal (2).

**6.** Dispositif médical (1) pour une cavité corporelle selon la revendication 1, dans lequel l'organe de fixation est un fil (4).

**7.** Dispositif médical (1) pour une cavité corporelle selon la revendication 1, dans lequel l'organe en forme de feuille (3) est fixé à la région du corps d'endoprothèse principal (2), avec un organe de fixation ayant un élément parallèle à la direction axiale de la région du corps d'endoprothèse principal (2).

**8.** Dispositif médical (1) pour une cavité corporelle selon la revendication 1, dans lequel au moins une partie du corps d'endoprothèse principal (2) a une structure pour la fixation de l'organe en forme de feuille (3).

**9.** Dispositif médical (1) pour une cavité corporelle selon l'une quelconque des revendications 1 à 8, dans lequel l'organe en forme de feuille (3) contient un matériau filamentaire imperméable aux rayons X au moins partiellement.

**10.** Dispositif médical (1) pour une cavité corporelle selon l'une quelconque des revendications 1 et 5 à 9, dans lequel l'organe en forme de feuille (3) contient un matériau imperméable aux rayons X au niveau du site de fixation.

**11.** Dispositif médical (1) pour une cavité corporelle selon l'une quelconque des revendications 2 à 4, dans lequel le dispositif médical (1) contient l'organe en forme de feuille (3) où les première et deuxième parties terminales de l'organe en forme de feuille (3) se chevauchent, lorsque la région du corps d'endoprothèse principal (2) présente le premier diamètre.

**12.** Dispositif médical (1) pour une cavité corporelle selon l'une quelconque des revendications 1 à 11, dans lequel l'organe en forme de feuille (3) est un maillage.

**13.** Dispositif médical (1) pour une cavité corporelle selon la revendication 6, dans lequel l'organe en forme de feuille (3) est un maillage et le maillage est formé par tissage d'un matériau filamentaire, et dans lequel le diamètre du fil (4) du matériau filamentaire de l'organe en forme de feuille (3) qui est le maillage est inférieur à l'épaisseur de la région du corps d'endoprothèse principal (2).

**14.** Dispositif médical (1) pour une cavité corporelle selon l'une quelconque de la revendication 12 ou 13, dans lequel la taille des pores de l'organe en forme de feuille (3) est inférieure ou égale à 0,2 mm.

**15.** Dispositif médical (1) pour une cavité corporelle selon l'une quelconque des revendications 1 à 14, dans lequel le matériau pour l'organe en forme de feuille (3) est un métal.

**16.** Dispositif médical (1) pour une cavité corporelle selon l'une quelconque des revendications 2 à 4, dans lequel la courbure de l'organe en forme de feuille (3) est différente de la courbure du corps d'endoprothèse principal (2) dans le deuxième diamètre.

**17.** Procédé de production du dispositif médical (1) selon l'une quelconque des revendications 6 à 16, comprenant une étape de couture consistant à permettre au fil (4) d'avoir un angle dans la région terminale, à placer l'organe en forme de feuille (3) à une position particulière, à envoyer le fil (4), lorsqu'il pénètre à travers l'organe en forme de feuille (3) et le corps d'endoprothèse principal (2) depuis le côté de surface externe jusqu'au côté périphérique interne du corps d'endoprothèse principal (2), et à envoyer la région terminale du fil (4) depuis le côté périphérique interne d'endoprothèse jusqu'au côté de surface externe en faisant avancer une tige (8) ayant un diamètre similaire au diamètre interne d'endoprothèse dans le côté périphérique interne du corps d'endoprothèse principal (2).

18. Système de pose, comprenant le dispositif médical (1) pour une cavité corporelle selon l'une quelconque des revendications 1 à 16 et un premier cathéter pour faire avancer le dispositif médical (1) pour une cavité corporelle dans le corps, où le dispositif médical (1) pour une cavité corporelle est situé dans la région distale du premier cathéter de sorte que le dispositif médical (1) puisse être positionné à demeure.

19. Système de pose selon la revendication 18, dans lequel au moins une partie de l'organe en forme de feuille (3) est placée séparément du corps d'endoprothèse principal (2), **caractérisé en ce que** le premier cathéter a un premier organe tubulaire (11), le corps d'endoprothèse principal (2) présentant un premier diamètre comprimé peut être placé intérieurement au niveau de l'extrémité distale du premier organe tubulaire (11), et l'organe en forme de feuille (3) peut être placé extérieurement au niveau de l'extrémité distale du premier organe tubulaire (11).

20. Système de pose selon la revendication 19, dans lequel le premier cathéter a un premier organe tubulaire (11) formant le corps principal de cathéter et un deuxième organe tubulaire (10) placé extérieurement sur le premier organe tubulaire (11), et le corps d'endoprothèse principal (2) présentant un premier diamètre comprimé peut être placé intérieurement au niveau de l'extrémité distale du premier organe tubulaire (11) et l'organe en forme de feuille (3) peut être placé entre les premier et deuxième organes tubulaires (10, 11).

21. Système de pose selon l'une quelconque des revendications 19 et 20, dans lequel le premier organe tubulaire (11) a une fente au niveau de l'extrémité distale.

22. Système de pose selon l'une quelconque des revendications 18 à 20, comprenant en outre un deuxième cathéter (13), pénétrant en étant inséré dans le premier organe tubulaire (11) de manière à pousser et pour une utilisation lors de la poussée du dispositif médical (1) pour une cavité de corps depuis le côté de bord arrière.

23. Système de pose selon l'une quelconque des revendications 18 à 22, dans lequel le premier cathéter ou le deuxième cathéter (13) conserve le diamètre interne en permettant l'absorption après le positionnement à demeure du dispositif médical.

[Fig. 1]

[Fig. 2]

[Fig. 3]

[Fig. 4]

[Fig. 5]

[Fig. 6]

[Fig. 7]

(a)

(b)

[Fig. 8]

[Fig. 9]

[Fig. 10]

[Fig. 11]

[Fig. 12]

[Fig. 13]

[Fig. 14]

(a)

(b)

(c)

[Fig. 15]

[Fig. 16]

[Fig. 17]

[Fig. 18]

[Fig. 19]

[Fig. 20]

(a)                                                (b)

Canine pathological sample (one month after stent indwelling)

[Fig. 21]

(a)                                        (b)

SEM photograph of aneurysmal phantom-sided region

[Fig. 22]

(a)

Photograph of aneurysmal phantom-sided region

(b)                                    (c)

SEM photograph of aneurysmal phantom-sided region

(d)                                    (e)

SEM photograph of synthetic blood vessel-sided region

[Fig. 23]

(a)

(b)

SEM photograph of aneurysmal phantom-sided region

(c)

(d)

SEM photograph of synthetic blood vessel-sided region

[Fig. 24]

(a)

Catheter     Aneurysm

0 minute after indwelling

(b)

30 minutes after indwelling

(c)

1 month after indwelling

[Fig. 25]

[Fig. 26]

[Fig. 27]

[Fig. 28]

[Fig. 29]

[Fig. 30]

[Fig. 31]

[Fig. 32]

(a)

(b)

(c)

(d)

(e)

(f)

[Fig. 33]

[Fig. 34]

[Fig. 35]

(a)

(b)

## REFERENCES CITED IN THE DESCRIPTION

### Patent documents cited in the description

- EP 0819412 A2
- JP 2001509412 A
- JP 2003175113 A
- JP 2001506176 A
- JP 8024346 A
- JP 2005052419 A
- JP 8224297 A
- EP 0716836 A1
- US 005779732 A